(19) Europäisches Patentamt

European Patent Office

Office européen des brevets

(11) **EP 1 657 312 A1**

(12) **EUROPEAN PATENT APPLICATION**

(43) Date of publication:
**17.05.2006 Bulletin 2006/20**

(51) Int Cl.:
***C12Q 1/68*** (2006.01)

(21) Application number: **05023684.3**

(22) Date of filing: **28.10.2005**

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br>Designated Extension States:<br>**AL BA HR MK YU** | • **de Longueville, Françoise**<br>**5360 Natoye (BE)**<br>• **Zammatteo, Nathalie**<br>**5024 Gelbressée (BE)**<br>• **Toussaint, Olivier**<br>**5020 Vedrin (BE)**<br>• **van Huffel, Christophe**<br>**1170 Watermael-Boitsfort (BE)** |
| (30) Priority: **12.11.2004 US 987212** | |
| (71) Applicant: **EPPENDORF AG**<br>**22339 Hamburg (DE)** | (74) Representative: **Becker Kurig Straus**<br>**Patentanwälte**<br>**Bavariastrasse 7**<br>**80336 München (DE)** |
| (72) Inventors:<br>• **Remacle, José**<br>**5020 Malonne (BE)** | |

(54) **Method and array for determination of RNAi cell transfection effects by multiple gene expression analysis on micro-arrays**

(57) The invention provides a tool and a method for the easy interpretation of the changes occurring in a cell, being a three dimensional complex and control system when transfected by RNAi. The method is based on the analysis of a limited number of data obtained by quantifying the intensity of the signals present on spots distributed in a two dimensional surface. The invention allows to observe the effects of the presence of a particular RNAI on the cells main vital cellular functions together with the possible side or deleterious effects resulting from the transfection process or to the presence of the RNAi in a cell. The invention also provides a method for the determination of change in the three dimensional status of a cell, wherein an array containing nucleic acids or proteins belonging to or being representative for at least 5 vital cellular functions together with 3 deleterious functions

**EP 1 657 312 A1**

## Description

### Field of the invention

The present invention relates to the field of analyzing gene expression changes occurring in cells when transfected by RNAi. The invention allows to observe the effects of the presence of a particular RNAI on the cells main vital cellular functions together with the possible side or deleterious effects resulting from the transfection process or to the presence of the RNAi in a cell. In particular, this invention pertains to a method and a kit for the quantitative determination of the change in the overall cellular status of a cell. In particular, this invention relates to a method for the determination of change in the three dimensional status of a cell, wherein an array containing nucleic acids or proteins belonging to or being representative for at least 5 vital cellular functions together with 3 deleterious functions , which functions being represented on the array by at least 20 genes or proteins, is contacted with a sample derived from a particular transfected cell , wherein the pattern obtained by the binding of the cell sample to the spots is indicative of the changes occuring in the cellular status.

### Description of the related art

The discovery that double-stranded RNA (dsRNA) molecules of 21-23 nt (short interfering RNAs, siRNAs) can silence targeted genes through sequence-specific cleavage of the cognate RNA transcript has led to the rapid adoption of technologies based on the RNA interference (RNAi) mechanism for analysis of gene function or to obtain a therapeutic effect. (Jackson et al., Nat Biotechnol 21(6) (2003), 635-7; Semizarov et al., Proc. Natl. Acad. Sci. USA 100(11) (2003), 6347-52; Chi et al., Proc. Natl. Acad. Sci. USA 100(11) (2003), 6343-6346).

Several methods have been proposed for the transfection of the RNAi in the cells. In US-P-6,506,559 a process of introducing dsRNA into a living cell is described, so as to inhibit gene expression of a target gene in that cell. Inhibition is sequence specific in that the nucleotide sequences of the duplex region of the RNA and of a portion of the target gene are identical.

In WO-02/44321 chemically synthesized siRNA duplexes of 19-25 nucleotides with overhanging 3'-ends are provided, to mediate efficient target mRNA cleavage.

In WO-03/006477 another method of inducing gene silencing is disclosed. Engineered dsRNA precursors are provided, which upon expression in a cell are processed by the cell to produce siRNAs that selectively silence targeted genes using the cell's own RNAi pathway.

US-A-20020173478 and 20030084471 show the applicability of RNAi to mammalian cells including human cells and cell lines, and propose these molecules for administration to human patients.

Other methods have been described for modulating RNAi pathway activity. In WO-01/29058 genes are provided, the expression products of which being involved in the mediation of RNAi. RNAi requires a set of conserved cellular factors to suppress gene expression. These factors are the components of the RNAi pathway (e.g. RDE-1, RDE-4) and provide activities necessary for interference. These activities may be absent or not sufficiently activated in many cell types to induce RNAi.

A critical assumption in the RNAi applications is that the effect of siRNA on cells is specific, i.e., limited to the specific knockdown of the target gene.

Three articles have been published which focussed on the efficacy and the specificity of the siRNA-induced gene silencing. They were performed on high-density arrays for wide expression profiling of cells transfected with different siRNAs against a single gene.

Jackson et al. (Nat Biotechnol 21(6) (2003), 635-7) have used gene expression profiling on high density microarrays containing 21,000 human probes to characterize the specificity of gene silencing by siRNAs in cultured human cells. Transcript profiles revealed siRNA-specific rather than target-specific signatures, including direct silencing of non-targeted genes containing as few as eleven contiguous nucleotides of identity to the siRNA. These results demonstrate that siRNAs may cross-react with targets of limited sequence similarity.

Semizarov et al. (Proc. Natl. Acad. Sci. USA 100(11) (2003), 6347-52) have characterized the specificity of siRNA by applying gene expression profiling on microarrays containing 12,000 genes and ESTs. Several siRNAs were designed against different regions of the same target gene for three different targets. Their effects on cells were compared by using DNA microarrays to generate gene expression signatures. When the siRNA design and transfection conditions were optimized, the signatures for different siRNAs against the same target were shown to correlate very closely, whereas the signatures for different genes revealed no correlation. These results indicate that siRNA is a highly specific tool for targeted gene knockdown, establishing siRNA-mediated gene silencing as a reliable approach for large-scale screening of gene function and drug target validation.

Chi et al. (Proc. Natl. Acad. Sci. USA 100(11) (2003), 6343-6346) have shown that siRNA-induced gene silencing of transient or stably expressed mRNA is highly gene-specific and does not produce secondary effects detectable by genomewide expression profiling on microarrays containing 43,000 elements, corresponding to 36,000 genes based on Unigene data.

These three works did no study of the side effects of the gene silencing mediated RNAi.

A vital assumption in the application of siRNA-mediated RNAi as a functional genomics tool is that the knockdown of a targeted gene is specific at both the RNA and protein level. Numerous reports in the literature described the exquisite specificity of siRNAs, suggesting a requirement for near-perfect identity with the siRNA sequence. However, most of the published analyses of siRNA-induced gene silencing have examined only one or a few genes in addition to the targeted gene. Cross-hybridization with transcripts containing partial identity to the siRNA sequence may elicit phenotypes reflecting silencing of unintended transcripts in addition to the target gene.

One potential complication in using siRNAs in genetic screening is that siRNAs are not always completely target-specific. Some "off-target" side effects have been reported, which in most cases can be attributed to partial homology of the un-intended target to the siRNA. Even though these off-target effects of RNAi are modest, compared with antisense RNA approaches, for example, it is often necessary to validate an identified phenotype caused by an siRNA with other independent siRNA directed against the same transcript. The amount of work is also extended by the number of sequences potentially efficient for getting the required inhibition of the gene of interest (Scacheri et al., Proc. Natl. Acad. Sci. USA **101(7)** (2004), 1892-1897). Several RNAi have to be tested to obtain the desired effect. Multi-RNAi screenings have been developed for this purpose. The US2004/0214181 proposes to use an array comprising multiple RNAi sequences immobilized on a support in order to screen for the most efficient sequence.

Also the presence of siRNA may lead to non desired or non-specific effects. siRNAs can non-specifically silence a subset of partially complementary mRNAs. A recent report shows that in addition to the well-known ability of siRNAs to silence specific genes, there is also a widespread non-specific effect on mammalian gene expression. Interestingly, expression of some genes was increased, whereas others were reduced (Persengiev et al., RNA 10 (2004), 12-18). The utility of RNAi had been limited by the innate immune response triggered by dsRNA; long dsRNAs induce the IFN response, which leads to the inhibition of protein translation by the PKR pathway, and activation of RNase L. These responses inhibit gene expression generally and significantly alter the cell physiology. However, although siRNAs are too short to induce the IFN response, the specificity of the gene silencing induced by siRNAs in mammalian cells has not been systematically examined. For instance, we do not know whether siRNAs can trigger additional antiviral mechanisms that significantly alter cell physiology, nor how the introduction of exogenous siRNAs might impact the processing and regulation of endogenous miRNAs and the genes that miRNAs control (Chi et al., Proc Natl Acad Sci USA 100(11) (2003), 6343-6346).

A recent report shows that in addition to the well-known ability of siRNAs to silence specific genes, there is also a widespread non-specific effect on mammalian gene expression. The non-specific effects observed raise the possibility that dsRNAs can affect gene expression through pathways other than PKR (like cell adhesion, metabolism, cytokine signalling, extracellular matrix) (Persengiev et al., RNA 10 (2004), 12-18). These non-specific effects must be considered in the design of siRNA mediated loss of function experiments.

siRNAs may also induce non-specific gene silencing through initiation of an antiviral response similar to the one that occurs following introduction of long (>30 bp) dsRNA--a response that is mediated through blockage of protein synthesis (via activation of protein kinase R and the subsequent phosphorylation of EIF2a) and non-specific RNA degradation (via activation of RNase L).

Other non-specific effects are related to the induction of IFN response genes although some times the IFN like effect is not exactly the same as in the presence of IFN (Bridge et al., Nat Genet 34(3) (2003), 263-4), to the apoptosis pathway (ex. p53 and p21) function in a vast number of related and unrelated cellular pathways, these genes would serve as highly sensitive detectors for direct and indirect off-target effects mediated by the siRNAs (Scacheri et al., Proc. Natl. Acad. Sci. USA **101(7)** (2004), 1892-1897).

The use of the high density chips, higher than 3000 for the study of the RNAi effect is confronted with the problem of having to handle huge amount of data to analyse some of them being not relevant to the question to be answered so that the tools is expensive and complicate and are not well adapted for screening purpose. They also are confronted to the problem of quantification in the measurement of the genes given the complexity of the arrays and the difficulty to optimize the probes individually.

Also the high density DNA micro-arrays commercially available carry several thousands of capture probes and are, therefore, due to this vast amount of sequences to be synthesized, purified, quantified, and to be fixed on the solid support quite expensive. Thus, although these "high-density" DNA micro-arrays may give to the basic researcher a means to identify potential novel mRNAs regulated in different tissues at different stages of their normal or abnormal development, they do not provide a data/price ratio high enough to to satisfy the researcher's desire for a tool applicable for routine analysis in their everyday research activities.

It was never described nor mentioned that it is possible to perform a quantitative study on low density microarrays for the detection of non specific side effects due to the presence of siRNA in a cell and not in association with the study of the main cell functions.

Thus there is a need for a method and a means to detect side effects induced by the presence of the RNAI in a cell.and to understand the complex regulatory relationships among large numbers of genes after inhibition of one of them by the presence of a given RNAi.

## Summary of the invention

The above problem has been solved by providing a method and tool for the determination and/or quantification of the effects of a siRNA transfection in cell(s) by the determination of non-specific RNAi effects, said method comprising the steps of: providing an array, having fixed on a substrate a maximum of 2999 different capture molecules specific of nucleic acids or proteins belonging to or representative for each of the following 3 non-specific effect functions: apoptosis, DNA repair/synthesis and interferon response wherein the array allows the detection of at least 20 different said nucleic acids or proteins, contacting a sample component derived from said transfected cell(s) with the array, detecting and/or quantifying the intensity of the signal on said capture molecules, comparing the transcriptome or proteome of said transfected cell(s) with at least one reference or control condition, wherein the pattern and/or the intensity of the binding events is indicative of the potentially deleterious non-specific side effects of the presence of siRNA in said transfected cell(s).

The present invention also provides a method and tool for the determination and/or quantification of the effects of a siRNA transfection in cell(s) by the determination of specific and non-specific RNAi effects in one assay, which method comprises the steps of providing an array, having fixed on a substrate a maximum of 2999 different capture molecules specific of nucleic acids or proteins belonging to or representative of each of the following 3 non-specific effect functions: apoptosis, DNA repair/synthesis and interferon response and at least one nucleic acid or protein, belonging to or representative of at least 4 of the following vital cellular functions: cell adhesion, cell cycle, growth factors and cytokines, cell signaling/receptor, chromosomal processing, intermediate metabolism, extracellular matrix, cell structure, protein metabolism, oxidative metabolism, transcription and housekeeping genes wherein the array allows the detection of at least 20 different said nucleic acids or proteins contacting a sample component derived from said transfected cell(s) with the array, detecting and/or quantifying the intensity of the signal on said capture molecules, comparing the transcriptome or proteome of said transfected cell(s) with at least one reference or control condition, wherein the pattern and/or the intensity of the binding events is indicative of the consequence of inhibition of a particular gene expression due to the presence of a siRNA on cellular vital functions and its potentially deleterious non-specific side effects of the presence of siRNA in said transfected cell(s).

In fact, the present invention allows to obtain a quantitative overview of the modifications occurring in cells via an analysis on a two dimensional surface of an array of the intensity of a limited number of signals correlated with gene expression or its products involved in or characteristic for at least 4 of the above mentioned main vital cellular functions. It has been found that this overall assessment of the at least 4 vital functions allows to reconstitute the relationships of these essential functions either between themselves or related to other specific functions.

The present invention preferably used arrays which contain capture molecules for the detection of the gene targeted by the siRNA.

### Brief Description of the Tables

Table 1 is a list of 93 genes on the array for determination of non-specific effect function for siRNA cell transfection effects. The table is organized in 3 pathways: apoptosis, DNA repair/synthesis and interferon response.

Table 2 is a list of 274 genes on the array classified according to their non-specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping genes)..

### Detailed Description of the Preferred Embodiments

**Definitions**

In the present invention, the term cell "vital function" or "vital cellular function" designates a cellular function which is essential for the life, division and growth of cells. Examples of such vital functions are in general selected from the group comprising, cell adhesion, cell cycle, growth factors and cytokines, cell signaling, chromosomal processing, intermediate metabolism, extracellular matrix, cell structure, protein metabolism, oxidative metabolism, transcription and housekeeping genes.

The term "expressed genes" are the parts of the genomic DNA which are transcribed into mRNA and then translated into a peptides or proteins. The measurement of the expressed genes is performed on either molecules within this process most currently the detection of the mRNA or of the peptide or protein. The detection can also be based on specific property of the protein being for example its enzymatic activity

The term "RNAi non-specific effect functions" are the cellular functions which are related to the apoptose, the DNA repair/synthesis and the interferon type response including some growth factors and cytokines; The list of some relevant gene of these functions is presented in table 1.

The term "expressed genes" are those regions of the genomic DNA which are transcribed into mRNA and optionally then translated into (poly-)peptides or proteins. According to the present invention the determination of expressed genes may be performed on either molecules, specifically via detection of the mRNA or of the (poly-)peptide or protein (which latter terms will be used hereinafter interchangeably). The determination may also be based on a specific property of the protein, e.g. its enzymatic activity.

The terms "nucleic acid, array, probe, target nucleic acid, bind substantially, hybridizing specifically to, background, quantifying" are as described in WO97/27317, which is incorporated herein by way of reference. In particular, the term "array" means a given number of capture probes being immobilized on support. The most common arrays are composed of capture probes being present in predetermined locations on a single support being or not a substrate for their binding. However, capture probes being present on multiple supports are also considered as arrays, as long as the different target molecules can be individually detected and/or quantified.

The term "nucleotide triphosphate" refers to nucleotides present in either DNA or RNA and thus includes nucleotides which incorporate adenine, cytosine, guanine, thymine and uracil as bases, the sugar moieties being deoxyribose or ribose. Other modified bases capable of base pairing with one of the conventional bases adenine, cytosine, guanine, thymine and uracil may be employed. Such modified bases include for example 8-azaguanine and hypoxanthine.

The term "nucleotide" as used herein refers to nucleosides present in nucleic acids (either DNA or RNA) combined with the bases of said nucleic acid, and includes nucleotides comprising usual or modified bases as above described.

References to nucleotide(s), polynucleotide(s) and the like include analogous species wherein the sugar-phosphate backbone is modified and/or replaced, provided that its hybridization properties are not destroyed. By way of example the backbone may be replaced by an equivalent synthetic peptide, called Peptide Nucleic Acid (PNA).

"Polynucleotide" sequences that are complementary to one or more of the genes described herein, refer to polynucleotides that are capable of hybridizing under stringent conditions to at least part of the nucleotide sequence of said genes. Polynucleotides also include oligonucleotides which can be used in particular conditions. Such hybridizable polynucleotides will typically exhibit at least about 75% sequence identity at the nucleotide level to said genes, preferably about 80% or 85% sequence identity or more preferably about 90% or 95% or more nucleotide sequence identity to said genes. They are composed of either small sequences typically 15-30 base long or longer ones being between 30 and 100 or even longer between 100 and 300 base long.

The terms "capture probe" relates to a molecule capable to specifically bind to a given polynucleotide or polypeptide. Polynucleotide binding is obtained through base pairing between two polynucleotides, one being the immobilized capture probe and the other one the target to be detected. Polypeptide binding is best performed using antibodies specific of the polypeptide for the capture of a given polypeptide or protein. Part of the antibodies, or recombinant proteins incorporating part of the antibodies, typically the variable domains, or even proteins being able to specifically recognize the peptide can also be used as capture probe. The term "capture probes" in the sense of the present invention shall designate genes or parts of genes of different lengths, e.g. between 10 and 1500 nucleotides, which are either synthesized chemically in situ on the surface of the support or laid down thereon. Moreover, this term shall also designate polypeptides or fragments thereof, or antibodies directed to particular polypeptides, which terms are used interchangeably, attached or adsorbed on the support.

The term "single capture nucleotide species" is a composition of related nucleotides for the detection of a given sequence by base pairing hybridization; nucleotides are synthesized either chemically or enzymatically but the synthesis is not always perfect and the main sequence is contaminated by other related sequences like shorter ones or sequences differing by one or a few nucleotides. The essential characteristic of one nucleotide species for the invention is that the overall species can be used for capture of a given sequence belonging to a given gene.

The "hybridized nucleic acids" are typically detected by detecting one or more "labels" attached to the sample nucleic acids. The labels may be incorporated by any of a number of means well known to those skilled in the art, such as detailed in WO 99/32660, which is incorporated herein by way of reference.

The present invention provides a method and tool for the analysis of differential expression of relevant key genes to understand the broad and complex side effects beyond the selective gene silencing of target genes when a siRNA molecule is introduced into cells. More specifically the invention allows the identification of the side effect of the RNAi on the apoptosis, interferon type response and DNA repair/synthesis pathways or activity.

The invention also provides a method and tools for the analysis of the genes of several pathways affected by a given specific gene inhibition due to the siRNA treatment. The invention gives with a limited number of data a visualization of essential pathways affected by the RNAi and of the siRNA specific action. More particularly, the invention provides an overall status/performance of a cell on the basis of the changes occuring in at least 4 cellular vital functions under the respective biological condition to analyze.

In one embodiment the invention allows to better design the sequence of the siRNA in order to obtain a better efficiency in the inhibition of a given gene.

In another embodiment, the invention allows to screen siRNA for their absence of non-specific or side effects, more particularly, to avoid siRNA reacting on other genes than the one of interest giving rise to false positive results.

None of the prior art documents mentions the necessity to investigate the different cellular functions related to the 3 non-specific effect functions (apoptosis, DNA repair/synthesis, interferon response) with a given restricted tool nor together with a determination of the vital cell functions and housekeeping gene quantification in order to provide a reliable and simple research or diagnostic tool.

In another embodiment, the present inventors provides a tool for the analysis of an overall status/performance of a cell on the basis of changes occurring in at least 4 vital functions considering the change in at least 2 functions among apoptosis, DNA repair/synthesis and interferon response of the particular cell.

According to another embodiment the present invention also provides a kit for carrying out the present methods, while the method and the kit being suitable for analyzing gene expression changes occurring in particular conditions a cell is subjected.

Accordingly, it has been found that in order to obtain a picture of a cellular status/performance essentially not all of the cellular genes/gene products have to be investigated but only some genes or gene products associated with the RNAi non-specific effect functions of the cell and optionally together with genes associated with vital functions of the cell. The main non-specific effect functions and the vital characteristic genes which have to be analyzed are described here after. The list of the main relevant genes associated with the 3 non-specific effect functions is given in table 1 and the overall gene list of both the non-specific effect functions and the vital functions are given in table 2.

## A) Non-specific effect functions

### 1. Apoptosis

Life requires death. Elimination of unwanted cells is vital for embryogenesis, metamorphosis and tissue turnover, as well as for the development and function of the immune system. For this reason, mammalian development is tightly regulated not only by the proliferation and differentiation of cells but also by cell death. The cell death that occurs during development or tissue turnover is called programmed cell death, most of which proceeds via apoptosis. Apoptosis is morphologically distinguished from necrosis, which occurs during the accidental cell death caused by physical or chemical agents. During apoptosis, the cytoplasm of the affected cells condenses, and the nucleus also condenses and becomes fragmented. At the final stage of apoptosis, the cells themselves are fragmented (apoptotic bodies) and are phagocytosed by neighboring macrophages and granulocytes.

Apoptosis or programmed cell death is triggered by a variety of stimuli, including cell surface receptors like FAS, the mitochondrial response to stress, and factors released from cytotoxic T cells. It constitutes a system for the removal of unnecessary, aged, or damaged cells that are regulated by the interplay of proapoptotic and antiapoptotic proteins of the Bcl-2 family. The proapoptotic proteins Bax, Bad, Bid, Bik, and Bim contain an $\alpha$-helical BH3 death domain that fits the hydrophobic BH3 binding pocket on the antiapoptotic proteins Bcl-2 and Bcl-$X_L$, forming heterodimers that block the survival-promoting activity of Bcl-2 and Bcl-$X_L$. Thus, the relative abundance of proapoptotic and antiapoptotic proteins determines the susceptibility of the cell to programmed death. The proapoptotic proteins act at the surface of the mitochondrial membrane to decrease the mitochondrial trans-membrane potential and promote leakage of cytochrome c. In the presence of dATP cytochrome c complexes with and activates Apaf-1. Activated Apaf-1 binds to downstream caspases, such as procaspase-9, and processes them into proteolytically active forms. This begins a caspase cascade resulting in apoptosis.

The caspases comprise a class of cysteine proteases many members of which are involved in apoptosis. The caspases convey the apoptotic signal in a proteolytic cascade, with caspases cleaving and activating other caspases that subsequently degrade cellular targets that lead to cell death. The activating caspases include caspase-8 and caspase-9. Caspase-8 is the initial caspase activated in response to receptors with a death domain that interacts with FADD. The mitochondrial stress pathway begins with the release of cytochrome c from mitochondria, which then interacts with Apaf-1, causing self-cleavage and activation of caspase-9. The effector caspases, caspase-3, -6 and-7 are downstream of the activator caspsases and act to cleave various cellular targets. Granzyme B and perforin, proteins released by cytotoxic T cells, induce apoptosis in target cells by forming transmembrane pores and triggering apoptosis, perhaps through cleavage of caspases. Caspase independent mechanisms of granzyme B-mediated apoptosis have been suggested.

According to the present invention 25 genes (or proteins, respectively) found to represent checkpoint genes in apoptosis were selected as preferred representatives of the apoptosis pathway. They comprise: BAD, BAX, BCL2, BCLX, BID, CAPS2, CASP3, CASP7, CASP8 and CASP9 ABL1, ADAM17, CASP1, CASP10, CYCS, E2F1, FADD, FASN, GSTT1, IGF2, IGFBP4, JUN, LTA, MAPK1, MAPK3 and are listed in table 1.

## 2. Interferon Response

One of the initial responses of an organism to infection by pathogenic viruses is the synthesis of antiviral cytokines such as the type I interferons (interferon-[alpha]/[beta]), interleukins, and other proinflammatory cytokines and chemokines. Interferons provide a first line of defense against virus infections by generating an intracellular environment that restricts virus replication and signals the presence of a viral pathogen to the adaptive arm of the immune response. Interferons stimulate cells in the local environment to activate a network of interferon-stimulated genes, which encode proteins that have antiviral, anti-proliferative and immunomodulatory activities.

Signaling by interferon-gamma stimulates anti-viral responses and tumor suppression through the heterodimeric interferon-gamma receptor. Signaling is initiated by binding of interferon-gamma to its receptor, activating the receptor-associated JAK2 tyrosine kinase to phosphorylate STAT transcription factors that activate interferon responsive genes. Molecular chaperones that modulate or alter protein folding interact with different components of the interferon signaling pathway. One chaperone that modulates interferon signaling is hTid-1, a member of the DnaJ family of chaperones and a co-chaperone for the heat shock protein Hsp70, another molecular chaperone. hTid-1 was found in a two-hybrid screen to bind to JAK2 and also to interact with the interferon-gamma receptor. In addition, hTid-1 and JAK2 also interact with Hsp70. Overexpression of hTid-1 represses transcriptional activation by interferon-gamma and Hsp70 dissociates from these proteins when interferon is added to cells, suggesting that Hsp70 holds Jak2 in an inactive conformation prior to ligand activation, and is released in the presence of agonist to allow the activation of Jak-2 and downstream pathways.

One of the actions of interferon is to induce apoptosis of infected target cells, in part through a mitochondrial dependent mechanism. An interaction between interferon signaling and Hsp70 may alter this mitochondrial apoptosis pathway, perhaps playing a role in interferon-mediated apoptosis of infected or transformed cells. The HTLV-1 Tax protein that interacts with Hsp70 blocks mitochondrial induced apoptosis, providing a protection against interferon-mediated cellular defenses.

According to the present invention 37 genes (or proteins, respectively) found to represent checkpoint genes in apoptosis were selected as preferred representatives of the Interferon reponse pathway (table 1). They comprise : B2M, C2, CNP, DUSP1, ENO1, ENPP1, FCER1G, G1P3, GEM, HLA-C, IFIT2, IFITM1, IFITM2, IFNAR1, IRF1, JAK1, JAK2, KLK3, MET, MX1, MYC, NRG1, NYREN18, OAS1, OAS2, OAS3, PLA2G1B, PML, PRAME, SH2D1A, SLC1A2, SPP1, STAT1, TAP1, TNFSF10, UBE2L6, WARS.

## 3. DNA Replication/repair

DNA replication in all species is very well regulated and is an extremely accurate process. In cells a large number of proteins play various critical roles to bring about replication. The central role in replication is played by the various DNA polymerases which have the task of synthesizing the new DNA strand one nucleotide at a time. Polymerase alpha (lagging strand replicase) Contains primase activity and has no proofreading 3'-5' exonuclease activity. Polymerase delta (leading strand replicase) lacks primase activity, has 3'-5' exonuclease activity. Proliferating cell nuclear antigen (PCNA) enhances processivity. DNA polymerases cannot replicate the extreme 5'-ends of chromosomes due to RNA priming (primer gap). The ends of chromosomes, or telomeres, consist of short repeated sequences that are synthesized by a polymerase called telomerase. Telomerase is a reverse transcriptase that adds de novo telomeric repeats onto chromosome ends or telomeres, compensating for the telomere loss that occurs due to the "end replication problem". Telomerase contains a catalytic subunit or TERT (telomerase reverse transcriptase) and an RNA component, Terc (telomerase RNA), which contains the sequence that telomerase uses as template for the addition of the new telomeric repeats and is therefore essential for enzyme activity. The maintenance of telomere length by telomerase is essential for chromosomal stability and cell viability and plays an important role in both tumor formation and aging. The loss of telomere repeats has been causally linked to replicative senescence by the demonstration that overexpression of the enzyme telomerase can result in the elongation or maintenance of telomeres and immortalisation of somatic cells with a diploid and apparently normal karyotype. Experimental evidence indicates that short telomeres accumulate prior to senescence and that replicative senescence is not triggered by the first telomere to reach a critical minimal threshold length. These observations are compatible with limited repair of short telomeres by telomerase-dependent or telomerase-independent DNA repair pathways. Deficiencies in telomere repair may result in accelerated senescence and aging as well as genetic instability that facilitates malignant transformation.

Unlike errors that occur during replication and are corrected by the DNA polymerases, damage to bases that occur post-replication are varied and occur in the life cycle of the cell. Agents that cause DNA damage include UV radiation which produces pyrimidine dimers in DNA. UV light-induced damage produces photoproducts that form cyclobutane (thymine-thymine) dimers.

The accurate transmission of genetic information relies mainly on the high fidelity of DNA replication, and the precise separation and equal distribution of chromosomes to the daughter cells. Besides that, cells have to deal with a wide variety of spontaneous DNA damage (for instance caused by reactive oxidative species) and induced DNA damage (by exposure with UV and ionising irradiation, and genotoxic compounds). To achieve this high fidelity of transmitting genetic information, cells have evolved a complex regulatory network of so called DNA damage checkpoint pathways, which are activated in the presence of DNA lesions as they may occur during DNA replication. The presence of a DNA lesion has to be recognized by a sensor, which transmits a signal via a network of signal transduction pathways to a series of downstream effectors molecules. Dependent on the origin of the DNA lesion, the cell type and the cell cycle stage, where the damage occurs, cells can respond to DNA lesions indifferent ways: (1) they trigger DNA repair to remove the damage (2) they transiently halt cell cycle progression or (3) if the damage is irreversible they induce cell death which leads to the removal of the harmed cell. Inherited or acquired deficiencies in DNA damage checkpoint pathways lead to genomic instability, which contributes significantly to the onset and progression of cancer (Christmann et al., Toxicology 193 (2003) 3-34; Sancar et al., Annu. Rev Biochem 73 (2004) 39-85).DNA damage checkpoint pathways can be described as signal transduction pathways (or as a network of interacting pathways) consisting of sensors, transducers and effectors. However a group of four conserved proteins are potential candidates for DNA damage sensors because they interact with DNA and they are essential for the activation of the DNA damage response pathways (reviewed in (O'Connell et al., Trends Cell Biol. **10(7)** (2000) 296-303)). In *S. pombe* three of them, Rad9, Hus1, and Rad1 form a heterotrimeric complex (9-1-1 complex) that shares homology with the sliding clamp protein proliferating cell nuclear antigen (PCNA).PCNA forms a ring wide enough to slide over double stranded DNA. In analogy to PCNA, it has been suggested that the 9-1-1 complex slides over DNA and continuously scans the genome for DNA damage. The fourth conserved checkpoint component is Rad17 (again using *S. pombe* nomenclature), which shares homology with subunits of the replication factor C (RFC). Rad17 acts upstream of the 9-1-1 complex and is thought to mediate the loading of the 1-1-9 complex onto DNA as it has been proposed for RFC that assembles PCNA on DNA (Figure 0-1). In addition, a repair complex is implicated in the sensing and signalling of DNA lesions. In *S. pombe* the repair complex Mre11-Rad50-Xrc2 (Human MRE11-RAD50-NBS1) is tethered to DNA ends of double strand breaks.

As part of an early DNA damage response two members of the large phosphatidylinositol-3-OH kinase family (PI3-K), *S. pombe Rad3*/hATR and S. *pombe Tel1*/hATM, are activated and recruited to sites of DNA damage. These DNA damage checkpoint kinase are thought to be the primary transducers of the DNA damage signal (reviewed in (Abraham, Genes & Dev 15 (2001), 2177-2196)). In the presence of DNA damage PI3-K are activated and trigger a signal transduction pathway by the phosphorylation of multiple DNA repair and DNA damage checkpoint proteins. Among these are the "downstream" protein kinases Cds1/hCHK2 and Chk1/hCHK1 (*S. pombe*/human nomenclature) that influence cell cycle progression differently dependent on the point in the cell cycle where the damage occurs (Matsuoka et al., Science 282 (1998), 1893-1897; Brown et al., Proc. Natl. Acad. Sci. USA 96 (1999), 3745-3750; Chaturvedi et al., Oncogene **18** (1999), 4047-4054).

Cell cycle delay in response to DNA damage is mainly regulated by the maintenance of the inhibitory phosphorylation of Cdk kinases. Cdks have to be dephosphorylated by the phosphatase Cdc25 to be active and to trigger cell cycle progression (Nurse, International Journal of Cancer 71 (1997), 707-708; Morgan D.O., Nature 374 (1995), 131-134). Upon DNA damage Cdc25 become inactivated. This inactivation is in part mediated by a Cds1/CHK2 and Chk1/CHK1 dependent phosphorylation of Cdc25 and its subsequent binding to 14-3-3 that lead to the retension of Cdc25 in the cytoplasm (Peng et al., Science 277(5331) (1997) 1501-5; Dalal et al., Mol. Cell. Biol. **19** (1999), 4465-4479; Yang et al., EMBO J. **18** (1999), 2174-2183). Beside a Cdc25 dependent cell cycle progression delay, mammalian cells induce a prolonged G 1 arrest by the activity of the tumour suppressor protein p53 (Levine A.J, Cell **88** (1997), 323-331). p53 is a sequence specific transcriptional regulator and induces the transcription of a variety of genes that are involved in cell cycle progression and apoptosis (Gottifredi et al., Cold Spring Harb Symp Quant Biol **65** (2000), 483-8). The transcriptional activity of p53 is mainly regulated by its turn over rate (reviewed in (Vogelstein et al., Nature **408** (2000), 307-310; Kastan et al., Cancer Research 51 (1991), 6304-6311)).DNA damage checkpoint activation induces increased p53 stability that leads to a transcriptional upregulation of cell cycle progression inhibitors (e.g. p21) that act by inhibiting Cdk kinase activity.

Response to DNA damage is not limited to cell cycle delay or apoptosis, but further involves control of DNA repair. Most DNA repair pathways are constitutively active, but a number of regulatory connections between DNA damage response pathways and DNA repair have been described (reviewed in (Lee and Kim, Mol.Cells 13(2) (2002), 15-166; D'Amours and Jackson, Nat. Rev. Mol. Cell Biol 3 (2002b), 317-327; Venkitaraman A.R., Cell 108 (2002), 171-182 ; Bartek et al., Nat. Rev. Mol. Cell Biol 2 (2001), 877-886)).For example, the p48 gene, which is involved in nucleotide excision repair, is transcriptionally upregulated by p53 (Hwang et al., Proc. Nat. Acad. Sci. 96 (1999), 424-428). Taken together, DNA damage activates complex signal transduction pathways that trigger cell cycle delay, apoptosis, and DNA repair.

In this invention, 31 genes (or proteins) are included into the array analysis as appropriate characteristic of the DNA replication and repair processes (table 1). They comprise : ADPRT, CROC1A, FHIT, GADD153, PLK, POLA2, RRM1, TERT, TOP2, TRF1, TYMS, BCL6, BRCA1, BRCA2, ERCC1, ERCC2, ERCC3, FEN1, GADD45A, MLH1, MSH2, PRKDC, RAD50, RAD51, RAD52, TNFRSF10B, TNFRSF4, TNFRSF6, UNG, XPA, XRCC1.

**Vital cellular functions**

**1. Cell adhesion**

Direct interactions between cells, as well as between cells and the extracellular matrix, are critical for the development and function of multicellular organisms. Some cell-cell interactions are transient, such as the interactions between cells of the immune system and the interactions that direct white blood cells to sites of tissue inflammation. In other cases, stable cell-cell junctions play a key role in the organization of cells in tissues. For example, several different types of stable cell-cell junctions are critical to the maintenance and function of epithelial cell sheets.

To form an anchoring junction, cells must first adhere. A bulky cytoskeletal apparatus must then be assembled around the molecules that directly mediate the adhesion. The result is a well-defined structure - a desmosome, a hemidesmosome, or an adherents or septate junction. In the early stages of development of a cell junction, however, before the cytoskeletal apparatus has assembled, and especially in embryonic tissues, the cells often adhere to one another without clearly displaying these characteristic structures. Many simple tissues, including most epithelia, derive from precursor cells whose progeny are prevented from wandering away by being attached to the extracellular matrix or to other cells or to both. But the cells, as they accumulate, do not simply remain passively stuck together as a disorderly pile; instead, the tissue architecture is actively maintained by selective adhesions that the cells make and progressively adjust. Thus, if cells of different embryonic tissues are artificially mingled, they will often spontaneously sort out to restore a more normal adhesion organization.

Cell-cell adhesion is a selective process, such that cells adhere only to other cells of specific types. Such selective cell-cell adhesion is mediated by transmembrane proteins called cell adhesion molecules, which can be divided into four major groups: the selectins, the integrins, the immunoglobulin (Ig), and the cadherins.

The selectins mediate transient interactions between leukocytes and endothelial cells or blood platelets. There are three members of the selectin family: L-selectin, which is expressed on leukocytes; E-selectin, which is expressed on endothelial cells; and P-selectin, which is expressed on platelets. The selectins recognize cell surface carbohydrates. One of their critical roles is to initiate the interactions between leukocytes and endothelial cells during the migration of leukocytes from the circulation to sites of tissue inflammation. The selectins mediate the initial adhesion of leukocytes to endothelial cells. This is followed by the formation of more stable adhesions, in which integrins on the surface of leukocytes bind to intercellular adhesion molecules (ICAMs), which are members of the Ig superfamily expressed on the surface of endothelial cells. The firmly attached leukocytes are then able to penetrate the walls of capillaries and enter the underlying tissue by migrating between endothelial cells.

The major cell surface receptors responsible for the attachment of cells to the extracellular matrix are the integrins. More than 20 different integrins, formed from combinations of subunits, have been identified. The integrins bind to short amino acid sequences present in multiple components of the extracellular matrix, including collagen, fibronectin, and laminin.

In addition to attaching cells to the extracellular matrix, the integrins serve as anchors for the cytoskeleton. The resulting linkage of the cytoskeleton to the extracellular matrix is responsible for the stability of cell-matrix junctions. Distinct interactions between integrins and the cytoskeleton are found at two types of cell-matrix junctions, focal adhesions and hemidesmosomes. Focal adhesions attach a variety of cells, including fibroblasts, to the extracellular matrix.

Cells dissociated from various tissues of vertebrate embryos preferentially reassociate with cells from the same tissue when they are mixed. This tissue-specific recognition process in vertebrates is mainly mediated by a family of $Ca^{2+}$-dependent cell-cell adhesion proteins called cadherins, which hold cells together by a homophilic interaction between transmembrane cadherin proteins on adjacent cells. In order to hold cells together, the cadherins must be attached to the cortical cytoskeleton. Most animal cells also have $Ca^{2+}$-independent cell-cell adhesion systems that mainly involve members of the immunoglobulin superfamily, which includes the neural cell adhesion molecule N-CAM. As even a single cell type uses multiple molecular mechanisms in adhering to other cells (and to the extracellular matrix), the specificity of cell-cell adhesion seen in embryonic development must result from the integration of a number of different adhesion systems, some of which are associated with specialized cell junctions while others are not.

In this invention, 10 genes (or proteins) are included into the array analysis as characteristic of the cell adhesion pathway (table 2). They comprise: CATB1, CDH1, CDH11, CDH13, ICAM1ITGA5, ITGA6, ITGB 1, TSP1 and TSP2.

**2. The Cell Cycle**

Cell division is the fundamental process by which all living organism grow, repair, and reproduce. In unicellular organisms, each cell division doubles the number of organisms; and in multicellular species, many rounds of cell division are required to produce a new organism or to replace cells lost by wear and tear or by programmed cell death.

In proliferating cells, the cell cycle consists of four phases. Gap 1 (G1) is the interval between mitosis and DNA replication that is characterized by cell growth. The transition that occurs at the restriction point (R) in G1 commits the cell to the proliferative cycle. If conditions that signal this transition are not present, the cell exits the cell cycle and enters G0, a nonproliferative phase during which growth, differentiation and apoptosis occur. Replication of DNA occurs during the synthesis (S) phase, which is followed by a second gap phase (G2) during which growth and preparation for cell division occurs. Mitosis and the production of two daughter cells occur in M phase.

Passage through the four phases of the cell cycle is controlled by a family of cyclins that act as regulatory subunits for cyclin-dependent kinases (cdks). The activity of the various cyclin/cdk complexes that regulate the progression through G1 -S-G2 phases of the cell cycle is controlled by the synthesis of the appropriate cyclins during a specific phase of the cell cycle. The cyclin/cdk complex is then activated by the sequential phosphorylation and dephosphorylation of the key residues of the complex, located principally on the cdk subunits.

The cyclin cdk complex of early G1 is either cdk2, cdk4, or cdk6 bound to a cyclin D isoform. There are several proteins that may inhibit the cell cycle in G1. If DNA damage occurred, p53 accumulates in the cell and induces the p21-mediated inhibition of cyclin D/cdk. Mdm2, by facilitating the nuclear export/inactivation of p53, becomes part of an inhibitory feedback loop that inactivates p21-mediated G1 arrest. Similarly, activation of TGF-$\beta$ receptors induces the inhibition of cyclin D/cdk by p15, while cyclic-AMP inhibits the cyclin D/cdk complex via p27.

If the cyclin D/cdk complex is inhibited, retinoblastoma protein (Rb) is in a state of low phosphorylation and is tightly bound to the transcription factor E2F, inhibiting its activity.

Passage through the restriction point and transition to S phase is triggered by the activation of the cyclin D/cdk complex, which phosphorylates Rb. Phoshporylated Rb dissociates from E2F, which is then free to initiate DNA replication. Cyclin E/cdk2 accumulates during late G phase and triggers the passage into S phase. The entire genome is replicated during S phase. The synthesis and accumulation of cyclin B/cdc2 also begins during S phase, but the complex is phosphorylated at $Thr^{14}$ -$Tyr^{15}$ and is inactive. Cyclin A/cdk2 accumulates during S phase and its activation triggers the transition to $G_2$, a phase characterized by the accumulation of cyclin B/cdc2, the inhibition of DNA replication, cell growth and new protein synthesis.

The transition from the $G_2$ phase to mitosis is triggered by the Cdc25-mediated activation (dephosphorylation) of the cyclin B/cdc2 complex (MPF). The activation of cyclin B/cdc2 that is necessary for G/M progression is currently the most well characterized step in the cell cycle. CyclinB/cdc2 is activated by phosphorylation of $Thr^{160}$ and the de-phosphorylation of $Thr^{14}$ - $Tyr^{15}$. $Thr^{160}$ is phosphorylated by cyclin activating kinase (CAK), following the activation of CAK by a cyclin activating kinase activating kinase (CAKAK). However, the complex is kept in an inactive state due to the phosphorylation of $Thr^{15}$, which is catalyzed by the Wee1 kinase. Cyclin B/cdc2 activation is triggered when Cdc25, a phospatase, dephosphorylates $Thr^{15}$. In turn, the activity of Cdc25 is regulated by both activating and inhibitory phosphorylations. Phosphorylation of Ser by Chk1 (a check point activated kinase that participates in the $G_2$-arrest of cells with damaged DNA) leads to the inactivation of Cdc25, while phosphorylation by an M-phase activated kinase creates a positive feedback loop leading to the rapid activation of the cyclin B/cdc2 complex.

MPF catalyzes the phosphorylation of lamins and histone 1, and is involved in the regulation of events preceding cell division, such as spindle formation, chromatin condensation, and fragmentation of the nuclear envelope and of organelles such as the Golgi and endoplasmic reticulum. The metaphase to anaphase transition is triggered by inactivation of MPF and the degradation of cyclin B. This induces the separation of chromatids and their movement to the poles of the mitotic spindle, after which the mitotic apparatus disappears, the nuclear membranes reform and the nucleoli reappear. During cytokinesis, the cytoplasm divides and the resulting daughter cells enter G1.

When cells traverse the $G_0$ to $G_1$ phase to the S-phase transition, a series of cyclin-dependent kinases is activated. The addition of serum growth factors to quiescent cells promotes transcription of the cyclin $D_1$ gene. Cyclin $D_1$ then associates with pre-existing cdk4 to form an active complex. The kinase activity associated with this complex can phosphorylate specific sites on the retinoblastoma protein (pRb), leading to inactivation of pRb and the activation cyclin E transcription by E2F. Activation of the cyclin E gene can be blocked by the cdk inhibitor p16. Cyclin E associates with existing cdk2 and this active complex regulates the function of several sets of target proteins. First, cyclin E/cdk2 complexes associate with E2F/p107 complexes to activate expression of the cyclin a gene. Also, cyclin E/cdk2 complexes cooperate with cyclin D 1 to amplify the phosphorylation of pRb. Cyclin A associates with cdk2 to form a kinase complex that phosphorylates downstream targets involved in the initiation of DNA replication.

In this invention, 32 genes (or proteins, respectively) have been selected as being good representatives of the cell cycle pathway being a vital function of the cell (table 2). They comprise : ATM, CAV1, CCNA1, CCNB1, CCND1, CCND2, CCND3, CCNE1, CCNF, CCNH, CDK2, CDK4, CDK6, DHFR, GRB2, MDM2, MKI67, p16, p21, p27, p35, p53, p57, PCNA, RB1, SMAD1, SMAD2, SMAD3, SMAD4, S100A4, S100A8, TK1.

### 3. Growth factors and cytokines

Growth factors are proteins that bind to receptors on the cell surface, with the primary result of activating cellular proliferation and/or differentiation. Many growth factors are quite versatile, stimulating cellular division in numerous different cell types, while others are specific to a particular cell-type.

Cytokines are a unique family of growth factors. Secreted primarily from leukocytes, cytokines stimulate both the humoral and cellular immune responses, as well as the activation of phagocytic cells. Cytokines that are secreted from lymphocytes are termed lymphokines, whereas those secreted by monocytes or macrophages are termed monokines. A large family of cytokines are produced by various cells of the body. Many of the lymphokines are also known as interleukins (ILs), since they are not only secreted by leukocytes but also able to affect the cellular responses of leukocytes. Specifically, interleukins are growth factors targeted to cells of hematopoietic origin.

EGF, like all growth factors, binds to specific high-affinity, low-capacity receptors on the surface of responsive cells. EGF has proliferative effects on cells of both mesodermal and ectodermal origin, particularly keratinocytes and fibroblasts. EGF exhibits negative growth effects on certain carcinomas as well as hair follicle cells. Growth-related responses to EGF include the induction of nuclear proto-oncogene expression, such as Fos, Jun and Myc.

Proliferative responses to PDGF action are exerted on many mesenchymal cell types. Other growth-related responses to PDGF include cytoskeletal rearrangement and increased polyphosphoinositol turnover. Again, like EGF, PDGF induces the expression of a number of nuclear localized proto-oncogenes, such as Fos, Myc and Jun.

There are at least 19 distinct members of the FGF family of growth factors. Studies of human disorders as well as gene knockout studies in mice show the prominent role for FGFs is in the development of the skeletal system and nervous system in mammals. Additionally, several members of the FGF family are potent inducers of mesodermal differentiation in early embryos. Non-proliferative effects include regulation of pituitary and ovarian cell function.

TGFs-b have proliferative effects on many mesenchymal and epithelial cell types. Under certain conditions TGFs-b will demonstrate anti-proliferative effects on endothelial cells, macrophages, and T- and B-lymphocytes. Such effects include decreasing the secretion of immunoglobulin and suppressing hematopoiesis, myogenesis, adipogenesis and adrenal steroidogenesis. Several members of the TGF-b family are potent inducers of mesodermal differentiation in early embryos, in particular TGF-b and activin A.

The predominant sources of TGF-$\alpha$ are carcinomas, but activated macrophages and keratinocytes (and possibly other epithelial cells) also secrete TGF-$\alpha$. In normal cell populations, TGF-$\alpha$ is a potent keratinocyte growth factor. The Mullerian inhibiting substance (MIS) is also a TGF-$\beta$-related protein, as are members of the bone morphogenetic protein (BMP) family of bone growth-regulatory factors.

IGF-I is a growth factor structurally related to insulin. IGF-I is the primary protein involved in responses of cells to growth hormone (GH): that is, IGF-I is produced in response to GH and then induces subsequent cellular activities, particularly on bone growth.

The predominant function of IL-1 is to enhance the activation of T-cells in response to antigen. The activation of T-cells, by IL-1, leads to increase T-cell production of IL-2 and of the IL-2 receptor, which in turn augments the activation of the T-cells in an autocrine loop. IL-1 also induces expression of interferon-g (IFN-$\gamma$) by T-cells. There are 2 distinct IL-1 proteins, termed IL-1-a and -1-b, that are 26% homologous at the amino acid level. The IL-1s are secreted primarily by macrophages but also from neutrophils, endothelial cells, smooth muscle cells, glial cells, astrocytes, B- and T-cells, fibroblasts and keratinocytes. Production of IL-1 by these different cell types occurs only in response to cellular stimulation. In addition to its effects on T-cells, IL-1 can induce proliferation in non-lymphoid cells.

IL-2, produced and secreted by activated T-cells, is the major interleukin responsible for clonal T-cell proliferation. IL-2 also exerts effects on B-cells, macrophages, and natural killer (NK) cells. The production of IL-2 occurs primarily by CD4+ T-helper cells. In contrast to T-helper cells, NK cells constitutively express IL-2 receptors and will secrete TNF-$\alpha$, IFN-g and GM-CSF in response to IL-2, which in turn activate macrophages.

IL-6 is produced by macrophages, fibroblasts, endothelial cells and activated T-helper cells. IL-6 acts in synergy with IL-1 and TNF-$\alpha$ (OK alpha something seems to be missing here) (in many immune responses), including T-cell activation. In particular, IL-6 is the primary inducer of the acute-phase response in liver. IL-6 also enhances the differentiation of B-cells and their consequent production of immunoglobulin. Unlike IL-1, IL-2 and TNF-$\alpha$, IL-6 does not induce cytokine expression; its main effects, therefore, are to augment the responses of immune cells to other cytokines.

IL-8 is an interleukin that belongs to an ever-expanding family of proteins that exert chemoattractant activity to leukocytes and fibroblasts. IL-8 is produced by monocytes, neutrophils, and NK cells and is chemoattractant for neutrophils, basophiles and T-cells. In addition, IL-8 activates neutrophils to degranulate.

TNF-$\alpha$, like IL-1 is a major immune response modifying cytokine produced primarily by activated macrophages. Like other growth factors, TNF-a induces expression of a number of nuclear proto-oncogenes as well as of several interleukins.

TNF-$\beta$ is characterized by its ability to kill a number of different cell types, as well as the ability to induce terminal differentiation in others. One significant non-proliferative response to TNF-$\beta$ is an inhibition of lipoprotein lipase present on the surface of vascular endothelial cells. The predominant site of TNF-β synthesis is T-lymphocytes, in particular the special class of T-cells called cytotoxic T-lymphocytes (CTL cells). The induction of TNF-b expression results from elevations in IL-2 as well as the interaction of antigen with T-cell receptors.

IFN-α, IFN-β and IFN-ω are known as type I interferons: they are predominantly responsible for the antiviral activities of the interferons. In contrast, IFN-γ is a type II or immune interferon. Although IFN-γ, has antiviral activity it is significantly less active at this function than the type I IFNs. IFN-γ is secreted primarily by CD8+ T-cells. Nearly all cells express receptors for IFN-γ and respond to IFN-γ binding by increasing the surface expression of class I MHC proteins, thereby promoting the presentation of antigen to T-helper (CD4+) cells. IFN-γ also increases the presentation of class II MHC proteins on class II cells further enhancing the ability of cells to present antigen to T-cells.

CSFs are cytokines that stimulate the proliferation of specific pluripotent stem cells of the bone marrow in adults. Granulocyte-CSF (G-CSF) is specific for proliferative effects on cells of the granulocyte lineage. Macrophage-CSF (M-CSF) is specific for cells of the macrophage lineage. Granulocyte-macrophage-CSF (GM-CSF) has proliferative effects on both classes of lymphoid cells. IL-3 (secreted primarily from T-cells) is also known as multi-CSF, since it stimulates stem cells to produce all forms of hematopoietic cells.

In this invention, 24 genes (or proteins) have been selected as appropriate representative of the growth factors and cytokines being a vital function of the cell (table 2). They comprise : BMP2, CSF1, CTGF, FGF2, FGF8, IGF1, IGFBP2, IGFBP3, IGFBP5, IL8, IL10, IL11, IL15, IL1A, IL1B, IL4, IL6, MEK1, MEK2, TNFA, VEGF, VEGFB, VEGFC and VEGFD.

## 4. Cell signaling/receptor

This is accomplished by a variety of signaling molecules that are secreted or expressed on the surface of a given cell and bind to receptors expressed by other cells, thereby integrating and coordinating the functions of the many individual cells that make up organisms as complex as human beings.

The binding of most signaling molecules to their receptors initiates a series of intracellular reactions that regulate virtually all aspects of cell behaviour, including metabolism, movement, proliferation, survival, and differentiation. Interest in this area is further heightened by the fact that many cancers arise as a result of a breakdown in the signaling pathways that control normal cell proliferation and survival.

Cells must be ready to respond to essential signals in their environment. These are often chemicals in the extracellular fluid (ECF) from: a) distant locations in a multicellular organism - endocrine signaling by hormones; b) nearby cells - paracrine stimulation by cytokines; c) even secreted by themselves (= autocrine stimulation).

They may also respond to molecules on the surface of adjacent cells (e.g. producing contact inhibition). Signaling molecules may trigger: a) an immediate change in the metabolism of the cell (e.g., increased glycogenolysis when a liver cell detects adrenaline); b) an immediate change in the electrical charge across the plasma membrane (e.g., the source of action potentials); c) a change in the gene expression - transcription - within the nucleus.

Two categories of signaling molecules (steroids and nitric oxide) diffuse into the cell where they bind internal receptors.

The others, e.g., proteins, bind to receptors displayed at the surface of the cell. These are transmembrane proteins whose extracellular portion has the binding site for the signaling molecule (the ligand); intracellular portion activates proteins in the cytosol that in different ways eventually regulate gene transcription in the nucleus.

They comprise: G-Protein-Coupled Receptors (GPCRs), Cytokine Receptors, Receptor Tyrosine Kinases (RTKs), JAK-STAT Pathways, Transforming Growth Factor-beta (TGF-b) Receptors, Tumor Necrosis Factor-a Receptors and the NF-kB Pathway, The T-Cell Receptor for Antigen (TCR)

In this invention, 15 genes (or proteins) have been selected as appropriate representatives of the cell signaling and receptor pathway being a vital function of the cell (table 2). They comprise : CSF1R, EGFR, ESR2, FGFR, IGF1R, IL11RA, NCK1, NCOR1, NCOR2, PGR, PLAUR, TBXA2R, VEGFR1, VEGFR2 and VEGFR3.

## 5. Chromosomal processing

In eukaryotic cells, the genetic material is organized in a complex structure composed of DNA and proteins, and is localized in the nucleus. This structure is called chromatin. In each cell, about two meters of DNA are contained in the nucleus. In addition to its high degree of compaction, DNA must be easily accessible to allow its interaction with the protein machinery allowing its replication, repair and recombination. The fundamental unit of chromatin is called nucleosome and is composed of DNA and histones. It constitutes the first level of compaction of DNA in the nucleus. This structure is regularly repeated to form nucleofilaments, which can adopt further compaction levels. The chromatin is divided in euchromatin and heterochromatin. The heterochromatin keeps the same structure along the cell cycle while the euchromatin appears less condensed during the interphase.

The histones H3, H4, H2A and H2B are very conserved basic proteins. They are the targets of numerous post-translational modifications, which could affect the accessibility to DNA and protein/protein interactions with the nucleosome.

The assembly of DNA into chromatin starts with the formation of tetramers (H3-H4)2 histones newly synthesized, which fix two dimers H2A-H2B (2). The newly synthesized histones are specifically modified, the most conserved modification being the acetylation of histone H4 on lysines 5 and 12. The maturation step requires ATP in order to allow a regular spacing of nucleosomes and the histones newly incorporated are desacetylated. The acetylation state results from equilibrium between two antagonist activities: the activity histone-acetyltransferase (HAT) and the activity histone-desacetylase (HDAC).

The chromatin can be submitted to variations at the DNA level (methylation) and at the histone level (post-translational modification, existence of variants such as CENPA, variant of histone 3). These modifications are able to induce difference in the structure and activity of chromatin. For instance, CENPA is associated to centromeric regions.

Chaperon histones may form complexes with histones to favor their assembly. For example, CAF-1 (Chromatin Assembly Factor 1) can react with acetylated histones H3 and H4 and take part to the assembly of chromatin and to the DNA replication. The interaction between CAF-1 and PCNA (Proliferating Cell Nuclear Antigen) establish a molecular bound between the chromatin assembly and the processes of DNA replication and repair.

In this invention, 6 genes (or proteins) have been selected as representative of the chromosomes processing being a vital function of the cell (table 2). They comprise : CENPF, H2B/S, H3FF, H4FM, KNSL5 and KNSL6.

**6. Intermediate metabolism**

The immediate source of energy for most cells is glucose but carbohydrates, fats and even proteins may in certain cells or at certain times be used as a source of energy (ATP).

Dietary carbohydrates from which humans gain energy enter the body in complex forms, such as disaccharides and the polymers starch (amylose and amylopectin) and glycogen. The first step in the metabolism of digestible carbohydrate is the conversion of the higher polymers to simpler, mono-saccharides soluble forms that can be transported across the intestinal wall and delivered to the tissues. The resultant glucose and other simple carbohydrates are transported across the intestinal wall to the hepatic portal vein and then to liver parenchymal cells and other tissues. There they are converted to fatty acids, amino acids, and glycogen, or else oxidized by the various catabolic pathways of cells. Oxidation of glucose is known as glycolysis. Glucose is oxidized to either lactate or pyruvate. Under aerobic conditions, the dominant product in most tissues is pyruvate and the pathway is known as aerobic glycolysis.

$$\text{Glucose} + 2\,\text{ADP} + 2\,\text{NAD}^+ + 2\,\text{Pi} \longrightarrow 2\,\text{Pyruvate} + 2\,\text{ATP} + 2\,\text{NADH} + 2\,\text{H}^+$$

When oxygen is depleted, as for instance during prolonged vigorous exercise, the dominant glycolytic product in many tissues is lactate and the process is known as anaerobic glycolysis.

Aerobic glycolysis of glucose to pyruvate requires two equivalents of ATP to activate the process, with the subsequent production of four equivalents of ATP and two equivalents of NADH. Thus, conversion of one mole of glucose to two moles of pyruvate is accompanied by the net production of two moles each of ATP and NADH.

The main enzymes involved in glycolysis are hexokinase, phosphohexose isomerase, phosphofructokinase-1, aldolase, glyceraldehyde-3-phosphate dehydrogenase, Phosphoglycerate kinase, enolase and pyruvate kinase.

Utilization of dietary lipids requires that they first be absorbed through the intestine. As these molecules are oils they would be essentially insoluble in the aqueous intestinal environment. Solubilization (emulsification) of dietary lipid is accomplished via bile salts that are synthesized in the liver and secreted from the gallbladder. The emulsified fats can then be degraded by pancreatic lipases (lipase and phospholipase A2). These enzymes, secreted into the intestine from the pancreas, generate free fatty acids and a mixture of mono- and diacylglycerols from dietary triacylglycerols. Following absorption of the products of pancreatic lipase by the intestinal mucosal cells, the resynthesis of triacylglycerols occurs. The triacylglycerols are then solubilized in lipoprotein complexes (complexes of lipid and protein) called chylomicrons. Triacylglycerols synthesized in the liver are packaged into VLDLs and released into the blood directly. Chylomicrons from the intestine are then released into the blood via the lymph system for delivery to the various tissues for storage or production of energy through oxidation.

Fatty acids must be activated in the cytoplasm before being oxidized in the mitochondria. Activation is catalyzed by fatty acyl-CoA ligase (also called acyl-CoA synthetase or thiokinase).

$$\text{Fatty acid} + \text{ATP} + \text{CoA} \longrightarrow \text{Acyl-CoA} + \text{PPi} + \text{AMP}$$

Oxidation of fatty acids occurs in the mitochondria. The process of fatty acid oxidation is termed b-oxidation since it occurs through the sequential removal of 2-carbon units by oxidation at the b-carbon position of the fatty acyl-CoA molecule. The oxidation of fatty acids yields significantly more energy per carbon atom than does the oxidation of carbohydrates. The main enzymes involved in the oxidation of fatty acids are acyl-CoA synthetase, enoyl-CoA hydratase, 3-hydorxyacyl-CoA dehydrogenase and thiolase.

One might predict that the pathway for the synthesis of fatty acids would be the reversal of the oxidation pathway. However, this would not allow distinct regulation of the two pathways to occur even given the fact that the pathways are separated within different cellular compartments. The pathway for fatty acid synthesis occurs in the cytoplasm, whereas, oxidation occurs in the mitochondria. The other major difference is the use of nucleotide co-factors. Oxidation of fats involves the reduction of FADH+ and NAD+. Synthesis of fats involves the oxidation of NADPH. Both oxidation and synthesis of fats utilize an activated two carbon intermediate, acetyl-CoA. However, the acetyl-CoA in fat synthesis exists temporarily bound to the enzyme complex as malonyl-CoA. The synthesis of malonyl-CoA is the first committed step of fatty acid synthesis and the enzyme that catalyzes this reaction, acetyl-CoA carboxylase (ACC), is the major site of regulation of fatty acid synthesis.

All tissues have some capability for synthesis of the non-essential amino acids, amino acid remodeling, and conversion of non-amino acid carbon skeletons into amino acids and other derivatives that contain nitrogen. However, the liver is the major site of nitrogen metabolism in the body. In times of dietary surplus, the potentially toxic nitrogen of amino acids is eliminated via transamination, deamination, and urea formation; the carbon skeletons are generally conserved as carbohydrate, via gluconeogenesis, or as fatty acid via fatty acid synthesis pathways. In this respect amino acids fall into three categories: glucogenic, ketogenic, or glucogenic and ketogenic. Glucogenic amino acids are those that give rise to a net production of pyruvate or TCA cycle intermediates, such as $\alpha$-ketoglutarate or oxaloacetate, all of which are precursors to glucose via gluconeogenesis. All amino acids except lysine and leucine are at least partly glucogenic. Lysine and leucine are the only amino acids that are solely ketogenic, giving rise only to acetylCoA or acetoacetylCoA, neither of which can bring about net glucose production. A small group of amino acids comprised of isoleucine, phenylalanine, threonine, tryptophan, and tyrosine give rise to both glucose and fatty acid precursors and are thus characterized as being glucogenic and ketogenic. Finally, it should be recognized that amino acids have a third possible fate. During times of starvation the reduced carbon skeleton is used for energy production, with the result that it is oxidized to $CO_2$ and $H_2O$.

In this invention, 6 genes (or proteins) are included into the array analysis as appropriate characteristic of the intermediate metabolism pathway (table 2). They comprise : CKB, ETFB, G6PD, GLB1, ODC and PKM2.

## 7. The Extracellular Matrix

The extracellular matrix (ECM) is a complex structural entity surrounding and supporting cells that are found within mammalian tissues. The ECM is often referred to as the connective tissue. The ECM is composed of 3 major classes of biomolecules:

1. Fibrous structural proteins: collagen and elastin.
2. Specialized proteins: e.g. fibrillin, fibronectin, and laminin.
3. Proteoglycans: these are composed of a protein core to which is attached long chains of repeating disaccharide units termed of glycosaminoglycans (GAGs) forming extremely complex high molecular weight components of the ECM.

The differences between the various types of extracellular matrix result from variations in the proportion of components.

The major structural protein of the extracellular matrix is collagen, which is the single most abundant protein in animal tissues. There are at least 12 types of collagen. Types I, II and III are the most abundant and form fibrils of similar structure. Type IV collagen forms a two-dimensional reticulum and is a major component of the basal lamina. Collagens are predominantly synthesized by fibroblasts but epithelial cells also synthesize these proteins.

Connective tissues also contain elastic fibbers, which are particularly abundant in organs that regularly stretch and then return to their original shape. Elastic fibbers are composed principally of a protein called elastin, which is crosslinked into a network by covalent bonds. This network of crosslinked elastin chains behaves like a rubber band, stretching under tension and then snapping back when the tension is released.

The role of fibronectin is to attach cells to a variety of extracellular matrices. Fibronectin attaches cells to all matrices except type IV that involves laminin as the adhesive molecule. Fibrillin is an integral constituent of the non-collagenus microfibrils of the extracellular matrix. The ECM includes Matrix MetalloProteinase (MMP).

In this invention, 16 genes (or proteins) are included into the array analysis as an appropriate characteristic of the extracellular proteins (table 2). They comprise : BSG, COL6A2, FN1, MMP 1, MMP9, MMP 11, MMP 12, MMP 13, MMP 14, MMP 15, MMP2, MMP3, MMP7, OPN, TIMP1 and TIMP2.

**8. Cell structure**

Cytoskeleton (intracellular)

Cellular responses to extracellular signals, including growth factors, frequently include changes in cell movement and cell shape. For example, growth factor-induced alterations in cell motility (as well as in cell proliferation) play critical roles in processes such as wound healing and embryonic development. In particular, many types of cell movement are based on the dynamic assembly and disassembly of actin filaments underlying the plasma membrane. Remodelling of the actin cytoskeleton therefore represents a key element of the response of many cells to growth factors and other extracellular stimuli.

A network of actin filaments and other cytoskeletal proteins underlies the plasma membrane and determines cell shape. Actin bundles also attach to the plasma membrane and anchor the cell at regions of cell-cell and cell-substratum contact.

Membrane cytoskeleton comprises the following proteins: microfilament (actin/myosin), spectrins (alpha, beta), dystrophin, ankyrin, adduxin, myosin, tropomyosin, dematin, glycophorin, fibronectin receptor, talin, vinculin, $\alpha$-actinin, fimbrin, villin, myosin I, spectrin, filamin, keratin, vimemtin, cytokeratin.

Microtubules are formed by the reversible polymerization of tubulin. They display dynamic instability and undergo continual cycles of assembly and disassembly as a result of GTP hydrolysis following tubulin polymerization.

Interior cytoskeleton comprises the following components: tubulin (microtubules), actin (microfilaments), stress fibers (microfilaments, myosin, a-actinin, tropomyosin, caldesmon), vinculin, talin, kinetochore, centrosome, spindle pole body, centriol, centractin, pericentrin.

Intermediate filaments are polymers of more than 50 different proteins that are expressed in various types of cells and can often identify the origin of the cell. They are not involved in cell movement, but provide mechanical support to cells and tissues. Intermediate filaments (IF) comprise: keratins, cytokeratins, nestin, vimentin, desmin, glial fibrillary acidic protein, peripherin, lamins. Intermediate filament-associated proteins (IFAP) comprise: epinemin, filaggrin, plectin, peripherin, restin, lamin.

Actin binding proteins (ABP) comprise : fragmin, b-actinin, gelsolin, villin, brevin, severin, filamin, spectrin, fodrin, $\alpha$-actinin, gelactin, fascin, vinculin, talin, fimbrin, tau, profilin, capping proteins.

In this invention, 7 genes (or proteins) are included into the array analysis as an appropriate characteristic of the cell structure proteins(table 2). They comprise : CDC42, EMS1, GSNON, PAK, SM22 and TB 10.

**9. Protein metabolism (synthesis/degradation)**

Protein synthesis

Translation is the RNA directed synthesis of polypeptides. This process requires all three classes of RNA. Although the chemistry of peptide bond formation is relatively simple, the processes leading to the ability to form a peptide bond are exceedingly complex. The template for correct addition of individual amino acids is the mRNA, yet both tRNAs and rRNAs are involved in the process. The tRNAs carry activated amino acids into the ribosome which is composed of rRNA and ribosomal proteins. The ribosome is associated with the mRNA ensuring correct access of activated tRNAs and containing the necessary enzymatic activities to catalyze peptide bond formation. Following assembly of both the small and large subunits onto the mRNA, and given the presence of charged tRNAs, protein synthesis can take place.

Translation proceeds in an ordered process. First accurate and efficient initiation occurs, then chain elongation and finally accurate and efficient termination must occur. All three of these processes require specific proteins, some of which are ribosome associated and some of which are separate from the ribosome, but may be temporarily associated with it.

Initiation of translation requires a specific initiator tRNA, tRNAmeti, that is used to incorporate the initial methionine residue into all proteins. The initiation of translation requires recognition of an AUG codon. A specific sequence context surrounding the initiator AUG aids ribosomal discrimination. This context is A/GCCA/GCCAUGA/G in most mRNAs. The specific non-ribosomally associated proteins required for accurate translational initiation are termed initiation factors.

The initiation factors eIF-1 and eIF-3 bind to the 40S ribosomal subunit favouring antiassociation to the 60S subunit. The prevention of subunit reassociation allows the preinitiation complex to form. The first step in the formation of the preinitiation complex is the binding of GTP to eIF-2 to form a binary complex. eIF-2 is composed of three subunits, a, b and g. The binary complex then binds to the activated initiator tRNA, met-tRNAmet forming a ternary complex that then binds to the 40S subunit forming the 43S preinitiation complex. The preinitiation complex is stabilized by the earlier association of eIF-3 and eIF-1 to the 40S subunit. The cap structure of eukaryotic mRNAs is bound by specific eIFs prior to association with the preinitiation complex. Cap binding is accomplished by the initiation factor eIF-4F. This factor is actually a complex of 3 proteins; eIF-4E, A and G. The protein eIF-4E is a 24 kDa protein which physically recognizes and binds to the cap structure. eIF-4A is a 46 kDa protein which binds and hydrolyses ATP and exhibits RNA helicase activity. Unwinding of mRNA secondary structure is necessary to allow access of the ribosomal subunits. eIF-4G aids in binding of the mRNA to the 43S preinitiation complex. Once the mRNA is properly aligned onto the preinitiation complex and the initiator met-tRNAmet is bound to the initiator AUG codon (a process facilitated by eIF-1) the 60S subunit associates with the complex. The association of the 60S subunit requires the activity of eIF-5 which binds first to the preinitiation complex. The energy needed to stimulate the formation of the 80S initiation complex comes from the hydrolysis of the GTP bound to eIF-2. The GDP bound form of eIF-2 then binds to eIF-2B which stimulates the exchange of GTP for GDP on eIF-2. When GTP is exchanged eIF-2B dissociates from eIF-2. This is termed the eIF-2 cycle (see diagram below). This cycle is absolutely required in order for eukaryotic translational initiation to occur. The GTP exchange reaction can be affected by phosphorylation of the a-subunit of eIF-2. At this stage the initiator met-tRNAmet is bound to the mRNA within a site of the ribosome termed the P-site, for peptide site. The other site within the ribosome to which incoming charged tRNAs bind is termed the A-site, for amino acid site.

The process of elongation, like that of initiation requires specific non-ribosomal proteins. Elongation of polypeptides occurs in a cyclic manner such that at the end of one complete round of amino acid addition the A site will be empty and ready to accept the incoming aminoacyl-tRNA dictated by the next codon of the mRNA. This means that not only does the incoming amino acid need to be attached to the peptide chain but also the ribosome must move down the mRNA to the next codon. Each incoming aminoacyl-tRNA is brought to the ribosome by an eEF-1a-GTP complex. When the correct tRNA is deposited into the A site the GTP is hydrolyzed and the eEF-la-GDP complex dissociates. In order for additional translocation events the GDP must be exchanged for GTP. This is carried out by eEF-1bg similarly to the GTP exchange that occurs with eIF-2 catalyzed by eIF-2B. The peptide attached to the tRNA in the P site is transferred to the amino group at the aminoacyl-tRNA in the A site. This reaction is catalyzed by peptidyltransferase. This process is termed transpeptidation. The elongated peptide now resides on a tRNA in the A site. The A site needs to be freed in order to accept the next aminoacyl-tRNA. The process of moving the peptidyl-tRNA from the A site to the P site is termed, translocation. Translocation is catalyzed by eEF-2 coupled to GTP hydrolysis. In the process of translocation the ribosome is moved along the mRNA such that the next codon of the mRNA resides under the A site. Following translocation eEF-2 is released from the ribosome. The cycle can now begin again.

Like initiation and elongation, translational termination requires specific protein factors identified as releasing factors. The signals for termination are termination codons present in the mRNA. There are 3 termination codons, UAG, UAA and UGA. The eRF binds to the A site of the ribosome in conjunction with GTP. The binding of eRF to the ribosome stimulates the peptidyltransferase activity to transfer the peptidyl group to water instead of an aminoacyl-tRNA. The resulting uncharged tRNA left in the P site is expelled with concomitant hydrolysis of GTP. The inactive ribosome then releases its mRNA and the 80S complex dissociates into the 40S and 60S subunits ready for another round of translation.

Protein degradation

The levels of proteins within cells are determined not only by rates of synthesis, but also by rates of degradation. The half-lives of proteins within cells vary widely, from minutes to several days, and differential rates of protein degradation are an important aspect of cell regulation. Many rapidly degraded proteins function as regulatory molecules, such as transcription factors. The rapid turnover of these proteins is necessary to allow their levels to change quickly in response to external stimuli. Other proteins are rapidly degraded in response to specific signals, providing another mechanism for the regulation of intracellular enzyme activity.

The major pathway of selective protein degradation in eukaryotic cells uses ubiquitin as a marker that targets cytosolic and nuclear proteins for rapid proteolysis. Ubiquitin is a 76-amino-acid polypeptide that is highly conserved in all eukaryotes. Proteins are marked for degradation by the attachment of ubiquitin to the amino group of the side chain of a lysine residue. Additional ubiquitins are then added to form a multiubiquitin chain. Such polyubiquinated proteins are recognized and degraded by a large, multisubunit protease complex, called the proteasome. Ubiquitin is released in the process, so it can be reused in another cycle. It is noteworthy that both the attachment of ubiquitin and the degradation of marked proteins require energy in the form of ATP.

Some enzymes involved in the degradation of proteins are: trypsin, trypsin inhibitors, chymotrypsin, proprotein convertase, cathepsins, kallikrein (hormone processing), calpain, metalloproteinases, hippostasin, granzyme, renin, elastase, C1 inhibitor.

In this invention, 6 genes (or proteins) are included into the array analysis as an appropriate characteristic of the protein metabolism (table 2). They comprise : ADAM1, CANX, CTSB, CTSDCTSL and EIF-4E.

**10. Oxidative metabolism**

Oxidative stress is imposed on cells as a result of one of three factors: 1) an increase in oxidant generation, 2) a decrease in antioxidant protection, or 3) a failure to repair oxidative damage. Cell damage is induced by reactive oxygen species (ROS). ROS are either free radicals, reactive anions containing oxygen atoms, or molecules containing oxygen atoms that can either produce free radicals or are chemically activated by them. Examples are hydroxyl radical, superoxide, hydrogen peroxide, and peroxynitrite. The main source of ROS *in vivo* is aerobic respiration, although ROS are also produced by peroxisomal β-oxidation of fatty acids, microsomal cytochrome P450 metabolism of xenobiotic compounds, stimulation of phagocytosis by pathogens or lipopolysaccharides, arginine metabolism, and tissue specific enzymes. Under normal conditions, ROS are cleared from the cell by the action of superoxide dismutase (SOD), catalase, or glutathione (GSH) peroxidase. The main damage to cells results from the ROS-induced alteration of macromolecules such as polyunsaturated fatty acids in membrane lipids, essential proteins, and DNA. Additionally, oxidative stress and ROS have been implicated in disease states, such as Alzheimer's disease, Parkinson's disease, cancer, and aging. In this invention, 4 genes (or proteins) are included into the array analysis as an appropriate characteristic of the oxidative metabolism (table 2). They comprise : SOD2, MSRA, GPX and GSTP1.

### 11. Transcription

The intricate task of regulating gene expression in the many differentiated cell types of multicellular organisms is accomplished primarily by the combined actions of multiple different transcriptional regulatory proteins. In addition, the packaging of DNA into chromatin and its modification by methylation impart further levels of complexity to the control of eukaryotic gene expression.

Despite the development of *in vitro* systems and the characterization of several general transcription factors, much remains to be learned concerning the mechanism of polymerase II transcription in eukaryotic cells.

Transcription/Transcription factors include:

- RNA polymerases, transcription factors, activator/ repressor
- STAT = signal transducer and activator of transcription, PIAS = protein inhibitor of activated STAT
- Homeobox / forkhead motif proteins, TATA-binding protein (TBP), SOX = family of SRY-related genes, which encode transcriptional factors involved in development. The Sox gene family consists of a large number of embryonically expressed genes related via the possession of a 79-amino-acid DNA-binding domain known as the HMG box.

Polycomb group (PcG) proteins were first described in Drosophila as factors responsible for maintaining the transcriptionally repressed state of Hox/homeotic genes in a stable and heritable manner throughout development. A growing number of vertebrate genes related to the Drosophila PcG proteins have recently been identified.

In this invention, 13 genes (or proteins) are included into the array analysis as abn appropriate characteristic of the transcription pathways (table 2). They comprise : DP1, DP2, E2F2, E2F3, E2F4, EGR1, EGR3, JUND, MAX, MYBL2, TFAP2A, TFAP2B and TFAP2C.

Beside some of the genes associated with the non-specific effect functions or vital functions some genes are also preferentially incorporated onto the chips detection for their roles in some specific cellular functions (cell differentiation, circulation, lipid metabolism, oncogenesis, tumor supressor, stress response, proteasome, housekeeping genes), some of them being possibly affected by the RNAi presence. Preferentially, the following genes are incorporated: SPRR1B, PAI1, PAI2, PLAU, TPA, VWF, ANX1, APOJ, COX2, c-myc, FES, FOS, RAF1, SHC, PSMD11, UBE2C, AOP2, HMOX, HSP27, HSP40, HSP70, HSP90-a, HSP90-b, JNK1, JNK2, JNK3, BIN1, ING1, TGFBR2, RPL13A, ALDOA, K-ALPHA-1, ACTB, PPIE, GAPD, HK1, HPRT1, MDH1, YWHAZ, RPS9, SDS, TFRC.

In a preferred embodiment, the array of the invention also comprises capture molecules for the detection of the gene targeted by the transfected siRNA.

According to a preferred embodiment, the step of detecting and optionally quantifying the intensity of signals is performed on a single capture nucleotide species and/or the values for the quantification on the arrays are taken as the average of three experimental data.

According to another preferred embodiment, the number of nucleic acids or proteins to be detected is at least of 50 and a maximum of 2999 and still preferentially of 1000. In preferred embodiment, the array of the invention comprises more than 50 different capture molecules and less than 1000.

In a specific embodiment, the "RNAiChips side effect" contains capture molecules for the detection of at least 20 and preferably 50 and still preferably 100 genes or all the genes according to the list provided in table 2. The array allows the simultaneous analysis of 274 different genes belonging to 3 different non-specific effect functions and to 11 other vital functions. Each gene detection is performed in triplicates. The value for the presence of each gene is the average of the three values and the mean is calculated together with the standard deviation. Each of the value is then corrected using internal standards which have been added in the analysis in a given concentration. Thereafter, a correction for housekeeping genes is also performed as an option for variations within the arrays. This process gives absolute values for the genes present in a test experiment compared to a control condition or to reference sample. The genes which are tested as significantly modified in a given experimental condition are presented in table 2.

According to a preferred embodiment the present methods are used to compare cellular conditions, wherein at least 2 and preferably 5 genes for each of the 3 non-specific effects functions are expressed differentially, said method further comprising the step of comparing the transcriptome of cells or tissues in a given biological condition with a reference or control condition.

According to another preferred embodiment at least 2 and preferably 5 genes of at least 4 vital cellular functions are expressed differentially together with at least 5 genes of a non-specific effect functions.

According to another preferred embodiment, the array comprises at least 20 and preferentially more than 50 different capture molecules with at least one capture molecule and preferably at least 5 capture molecules for each of the 3 non-specific effect functions.

According to another preferred embodiment, the array comprises at least 5 different capture molecules for each of the 3 non-specific effect functions and at least 5 and more preferably 20 different capture molecules of at least 4 vital cellular functions.

According to a preferred embodiment, the 3 non-specific effect functions on the array are represented by at least 1 gene per function and preferably 5 genes per function from table I. The vital functions of a cell may be any function that fulfill the above definition. Yet, according to a preferred embodiment each of the vital functions on the array are represented by at least 5 genes of table 2, and more preferably by 20 genes derived from table 2.

According to another preferred embodiment at least one gene for each of the 11 vital functions is a gene which affects a regulatory activity in the function.

The cell to be investigated may be any prokaryotic or eucaryotic cell but is preferably a cell selected from the group consisting of cardiomyocytes, endothelial cells, sensory neurons, motor neurons, CNS neurons, astrocytes, glial cells, Schwann cells, mast cells, eosinophils, smooth muscle cells, skeletal muscle cells, pericytes, lymphocytes, tumor cells, monocytes, macrophages, foamy macrophages, dentritic cells, granulocytes, melanocytes, keratinocytes, synovial cells/synovial fibroblasts and epithelial cells.

The array to be used may be any conventional "biochip structure" means a support or a subtrate present on a support on which are bound capture molecules. The area where a target is seen by the method of detection is a localized area. It represents the area of the capture molecules specific of this target.

According to one embodiment the array comprises capture molecules being polynucleotides and/or peptides, antibodies or related proteins having specific affinity for proteins present on a solid substrate.

The capture molecules are preferentially bound to the surface of the substrate or on the support by physical deposition of a solution containing the capture molecules in different areas of the substrate. In another embodiment, capture molecules are synthetized in situ on the substrate in predefined locations. In another embodiment the capture molecules are present in defined locations on the substrate.

According to a preferred embodiment of the invention capture molecules are long polynucleotides and are uniques for each of the genes to be detected and/or quantified on the array. Long capture probes mean capture probes of 15 to 1000 nucleotides in length, e.g. of 15 to 200, or 15 to 150 nucleotides or 15 to 100, and are fixed on a support being any solid support as long as they are able to hybridized with their corresponding cDNA and be identified and quantified. The amount of the capture nucleotide sequences bound to the surface of the solid support is superior to 3 finoles per cm2 of solid support surface.

In a preferred embodiment, the sample component derived from the transfected cell(s) is RNA transcripts or nucleic acids derived from said RNA transcripts. A nucleic acid derived from an mRNA transcript refers to a nucleic acid for whose synthesis the mRNA transcript or a subsequence thereof has ultimately served as a template. Thus, a cDNA reverse transcribed from an mRNA, an RNA transcribed from that cDNA, a DNA amplified from the cDNA, an RNA transcribed from the amplified DNA, are all derived from the mRNA transcript and detection of such derived products is indicative of the presence and/or abundance of the original transcript in a sample. Direct capture of the cDNA is the preferred embodiment making the data mining and the interpretation of the results easier. The method does not exclude the use of fragmented cDNA or RNA as a means of detection of gene by determining for each gene the pattern of hybridization on polynucleotides present on the array.

In another embodiment, the capture probes bind the cDNA of the genes close to the 3 poly(A+) region of the corresponding mRNA. Retro-transcription of mRNAs begin at their 3'-poly(A+) region and is not always complete, which indeed gives rise to a population of more or less complete cDNAs. To improve the efficiency of hybridization, it may thus appear preferable to design capture sequences specific to mRNAs regions close to their 3'-end.

The capture probes sequences are preferably chosen so as to avoid cross-reaction with other gene(s). Therefore, regions of high (>50%) homologies between genes, able to yield cross-hybridizations between capture probes and target cDNAs will not be the preferred species.

Also variants of the genes may specifically be detected and quantified on the array by specific capture probes. In some tissues, multiple mRNAs are transcribed from the same gene. These variants often exhibit more or less overlapping sequences. Selection of the capture probes has to be specific for such marker, when all variants do not have the same potential importance as diagnostic, prognostic, or predictive indicator, or when it is recommended to detect only some specific ones.

According to an embodiment the method may be performed on cDNA obtained by retro-transcription from total RNA or mRNA. To this end, total RNA is extracted from tissue and an amount of about 0.1 to 100 $\mu$g, preferably 0.1 to 50 $\mu$g, more preferably 0.1 to 20 $\mu$g, even more preferably 0.1 to 10 $\mu$g, or even more preferred between 0.1 and 2 $\mu$g is used for direct labeling and hybridization on the array. Messenger RNA may also be processed in the same way with a much lower amount to be used for the copying into cDNA. When RNA is amplified by T7 polymerase based method, PCR, rolling circle or other methods, detection is possible even at lower concentration than 0.1 $\mu$g of total RNA or mRNA depending on the amplification obtained being usually in the order of a few hundreds for the T7 polymerase and much higher for the PCR or rolling circle amplifications. In extreme cases, detection of a single cell or a group of a few cells like obtained by laser dissection methods is feasible. In amplification methods, however, different genes are amplified with different efficiencies and corrections have to be provided for the pitfall introduced in the differentially amplified genes.

According to another preferred embodiment the original nucleotide sequences to be detected and/or to be quantified are RNA sequences by the retro-transcription of the 3' or 5' end wherein consensus primer and possibly a stopper sequence are used. Preferably, the copied or amplified sequences are detected without previous cutting of original sequences into smaller portions.

According to a preferred embodiment the sample and the control experimental conditions are analyzed on the same support having two identical arrays. In another embodiment, the sample to be tested and the control are analyzed on arrays bearing the same capture molecules for the said analyzed genes.

According to a preferred embodiment, the measurement on the array of the genes expressed in the cells are quantitative with coefficient of variation of the data lower than 80% even lower than 25% even lower than 15%.

The present invention also pertains to a diagnostic and/or prognostic kit, which comprises means and media for performing the above method.

According to a preferred embodiment, the present invention provides a kit for determination and/or quantification of the effects of a siRNA transfection in cell(s) by the determination of non-specific RNAi effects, comprising: an array, having fixed on a substrate a maximum of 2999 different capture molecules specific of nucleic acids or proteins belonging to or representative for each of the following 3 non-specific effect functions: apoptosis, DNA repair/synthesis and interferon response wherein the array allows the detection of at least 20 different said nucleic acids or proteins, and optionally buffers and labels.

According to another preferred embodiment the present invention provides a kit for determination and/or quantification of the effects of siRNA transfection in cell(s) by the determination specific and non-specific RNAi effects, comprising : an array, having fixed on a substrate a maximum of 2999 different capture molecules specific of nucleic acids or proteins belonging to or representative of each of the following 3 non-specific effect functions: apoptosis, DNA repair/synthesis and interferon response and at least one nucleic acid or protein, belonging to or representative of at least 4 of the following vital cellular functions: cell adhesion, cell cycle, growth factors and cytokines, cell signaling/ receptor, chromosomal processing, intermediate metabolism, extracellular matrix, cell structure, protein metabolism, oxidative metabolism, transcription and housekeeping genes wherein the array allows the detection of at least 20 different said nucleic acids or proteins, and optionally buffers and labels.

Alternatively, the method and kit of the present invention may be used for the determination of changes in gene expression occurring in particular conditions a cell is subjected in the presence of siRNA. Proceeding accordingly will allow to e.g. elucidate the role of particular genes in a given physiological event, such as stress, ageing, stem cell differentiation, haematopoiesis, neuronal functional status, diabetes, obesity, transformation process such as carcinogenesis, protein turnover or circulatory disorders as atherosclerosis.

In another embodiment the method and kit of the invention are used for better designing the sequence of the transfected siRNA in order to obtain a better efficiency in the inhibition of a given gene.

In another embodiment the method and kit of the invention are used to screen siRNA for their absence of non-specific or side effects, more particularly, to avoid siRNA reacting on other genes than the one of interest giving rise to false positive results. In other words, the invention allows to screen for siRNAi having high efficiency of effect on a particular gene or pathways ; also the invention allows to screen for siRNA having low side effects.

In a preferred embodiment, the method and kit of the invention are used for the screening for an agent capable of modulating the effect of a siRNA, comprising the steps of exposing a cell to the agent; and detecting the expression level of at least one gene associated with each of at least 2 of the 3 non-specific effect functions and at least 10 other genes associated with at least 3 vital functions as listed in table 2.

In another embodiment, the method and kit of the invention are used for the screening for an agent capable of modulating the effect of siRNA transfection in cell(s), comprising the steps of exposing a transfected cell(s) to the agent; detecting the expression level of at least one gene associated with each of at least 2 of the 3 non-specific effect functions and at least 10 other genes associated with at least 3 vital functions as listed in table 2, and comparing the transcripome or proteome of the transfected cell(s) with at least one reference cell(s). Reference cell(s) may be non transfected cell(s) contacted with the agent or transfected cell(s) which were not contacted with the agent.

The invention is particularly useful to follow RNAi effects in the presence of agents selected from the group consisting of biological molecules such as cytokines, growth hormones, or any biological molecules affecting cells. It also comprises chemical compounds such as drugs, toxic molecules, compounds from plants or animal extracts, chemicals resulting from organic synthesis including combinatory chemistry.

In another embodiment, the invention further includes computer systems comprising a database containing information identifying the expression level of cell(s) transfected with siRNA and genes associated with each of the 3 non-specific effect functions and with the 11 cellular vital functions as listed in tables -1-2 and a user interface to view the information. The present invention includes relational databases containing sequence information, for instance for one or more of the genes of tables 1-2, as well as gene expression information for various siRNA transfected cell(s). Databases may also contain information associated with a given siRNA sequence such as descriptive information about the gene associated with the sequence information. The database may be designed to include different parts, for instance a sequence database and a gene expression database. Methods for the configuration and construction of such databases are widely available, for instance, US-5,953,727, which is incorporated herein by reference in its entirety. In another embodiment, the genes detected as associated with a function are regulated on the transcriptional level.

The genes as mentioned here to be detected on the array may be derived from public databases (i.e. the gene ontology project at http://www.geneontology.org/).

The following example illustrates the invention without limiting it thereto.

## EXAMPLE

**Detection of gene expression : example of siRNA (Bcl-2) cell transfection effects.**

*Preparation of the siRNA related to the Bcl-2 gene*

The preparation of dsRNA was performed using the T7 RiboMAX Express RNAi system (Promega, Madison, WI, USA).

The cloned Bcl-2 gene was amplified with specific primers (5'-AGCACAGAAGATGGGAACAC-3', and 5'-CAGCCACCTCTTAAAAGTATC-3'), one of them having a T7 promoter of the type 5'-TAATACGACTCACTATAGGN (primer) 3'.

The resulting amplicons had a T7 promoter on one strand thus allowing the transcription of this strand. The transcription was performed with the RiboMAX Express as proposed by the manufacturer by incubating each of the T7 stranded sequences with the transcriptase. The reaction was performed at 37°C for 30 min. After transcription, the DNA template is removed by digestion with DNase, being RNase free. The annealing of the two RNA strands was obtained by incubating an equal concentration and volume of the two RNA strands for 10 min at 70°C. The single stranded RNA was then removed by incubation with RNase which digests the single stranded RNA. The double stranded RNA was then purified by precipitation in cold ethanol (- 70°C) and recovered after centrifugation in solution ready for digestion with the Silencer siRNA cocktail kit of Ambion (Austin, TX USA). The double stranded RNA was then digested using 1 U of RNase III per microgram of RNA with incubation of 1 h at 37°C. After one hour digestion, the double stranded RNA was reduced into small fragments of lower than 30 bp with a majority of between 12 and 15 bp.

*Transfection*

The siRNA was transferred in the wells of a 96 well plate and CHO cells were transferred into the wells with the siPort Amine Transfection Agent of Ambion (Austin, Tx., USA) using the experimental protocol provided by the manufacturer with a concentration of 3 $\mu$l of transfection agent per cm$^2$ of plate surface and a density of cells being at $10^5$ cell per cm$^2$. Control cells were non transfected cells incubated in the same conditions.

After 2 days the mRNA of the cells was collected and the level of expression of the Bcl-2 genes tested on the microarray of the invention.

The level of the mRNA coding for Bcl-2. was shown by RT-PCR as being lower in the cells which have been transfected in the wells containing the siRNA specific of the Bcl-2 sequence as compared to the non transfected cells.

*Gene expression detection on microarrays*

1. RNA extraction:

Poly(A$^+$) RNA (mRNA) was extracted using FastTrack columns (InVitrogen). Poly(A+) RNA was resuspended in RNAse-free water.

20

The concentration and purity of RNA was determined by diluting an aliquot of the preparation in TE (10mM Tris-HCl pH 8, 1mM EDTA) and measuring (reading) its absorbance (in a spectrophotometer) at 260 nM and 280 nm.

While the A260 value allows to evaluate the RNA concentration, the A260/A280 ratio gives an indication of the RNA purity. For a RNA to be used, its ratio must be between 1.8 and 2.

The overall quality of the RNA preparation was determined by electrophoresis on a denaturing 1% agarose gel (Sambrook et al., eds. (1989) Molecular Cloning — A Laboratory Manual, 2nd ed. Cold Spring Harbor, NY: Cold Spring Harbor Laboratory Press).

2. cDNA synthesis:

1 $\mu$l of poly(A$^+$) RNA sample (0.5 $\mu$g/$\mu$l) was mixed with 2 $\mu$l oligo(dT)$_{12\text{-}18}$ (0.5 $\mu$g/$\mu$l, Roche), 3.5 $\mu$l H$_2$O, and 3 $\mu$l of a solution of 3 different synthetic well-defined poly(A+) RNAs. These latter served as internal standards to assist in quantification and estimation of experimental variation introduced during the subsequent steps of analysis. After an incubation of 10 minutes at 70°C and 5 minutes on ice, 9 $\mu$l of reaction mix were added. Reaction mix consisted in 4 $\mu$l Reverse Transcription Buffer 5X (Gibco BRL), 1 $\mu$l RNAsin Ribonuclease Inhibitor (40 U/ml, Promega), and 2 $\mu$l of a 10X dNTP mix, made of dATP, dTTP, dGTP (5 mM each, Roche), dCTP (800 $\mu$M, Roche), and Biotin-11-dCTP (800 $\mu$M, NEN).

After 5 min at room temperature, 1.5 $\mu$l SuperScript II (200 U/ml, Gibco BRL) was added and incubation was performed at 42°C for 90 minutes. Addition of SuperScript and incubation were repeated once. The mixture was then placed at 70°C for 15 minutes and 1 $\mu$l Ribonuclease H (2U/$\mu$l) was added for 20 minutes at 37°C. Finally, a 3-min denaturation step was performed at 95°C. The biotinylated cDNA, was kept at -20°C.

3. Hybridization of (using) biotinylated cDNA:

The array used for the hybridization of biotinylated cDNA comprises 274 genes (table 2) and several different controls including positive and negative detection control, positive and negative hybridization control, three different internal standards all dispersed at different locations among the genes to be analyzed on the micro-array. The array comprises the Bcl-2 capture probe which is used to determine siRNA specific effect (on Bcl-2 target gene). The 93 genes belonging to the apoptosis, DNA repair/synthesis and IFN response pathways are used to determine non-specific siRNA effect (off Bcl-2 target gene).

Each spots was covered with a capture probe being a polynucleotide species, which allow the specific binding of one target polynucleotide, corresponding to a specific gene listed in table 1. All sequences have been designed to be gene specific and have been prepared using human cDNA clones.

Hybridization chambers were from Biozym (Landgraaf, The Netherlands). Hybridization mixture consisted in biotinylated cDNA (the total amount of labeled cDNA), 6.5 $\mu$l HybriBuffer A (Eppendorf, Hambourg, Germany), 26 $\mu$l HybriBuffer B (Eppendorf, Hambourg, Germany), 8 $\mu$l H$_2$O, and 2 $\mu$l of positive hybridization control.

Hybridization was carried out overnight at 60°C. The micro-arrays were then washed 4 times for 2 min with washing buffer (Eppendorf, Hamburg, Germany).

The micro-arrays were than incubated for 45 min at room temperature with the Cy3-conjugated IgG Anti-biotin (Jackson Immuno Research laboratories, Inc #200-162-096) diluted 1/1000 X Conjugate-Cy3 in the blocking reagent and protect from light.

The micro-arrays were washed again 4 times for 2 minutes with washing buffer and 2 times for 2 minutes with distilled water before being dried under a flux of N$_2$.

4. Scanning and data analysis:

The hybridized micro-arrays were scanned using a laser confocal scanner "ScanArray" (Packard, USA) at a resolution of 10 $\mu$m. To maximize the dynamic range of the assay the same arrays were scanned at different photomultiplier tube (PMT) settings. After image acquisition, the scanned 16-bit images were imported to the software, "ImaGene4.0" (BioDiscovery, Los Angeles, CA, USA), which was used to quantify the signal intensities. Data mining and determination of significantly expressed gene in the test compared to the reference arrays was performed according to the method described by Delongueville et al (Biochem Pharmacol. 64(1) (2002),137-49). Briefly, the spots intensities were first corrected for the local background and than the ratios between the test and the reference arrays were calculated. To account variation in the different experimental steps, the data obtained from different hybridizations were normalized in two ways. First the values are corrected using a factor calculated from the intensity ratios of the internal standard reference and the test sample. The presence of 3 internal standard probes at different locations on the micro-array allows measurement of a local background and evaluation of the micro-array homogeneity, which is going to be considered in the normalization (Schuchhardt et al., Nucleic Acids Res. 28 (2000), E47). However, the internal standard control does not account for the quality of the mRNA samples, therefore a second step of normalization was performed based on the expression levels of housekeeping genes. This process involves calculating the average intensity for a set of housekeeping genes, the expression of which is not expected to vary significantly. The variance of the normalized set of housekeeping genes is used to generate an estimate of expected variance, leading to a predicted confidence interval for testing the significance of the ratios obtained (Chen et al, J. Biomed. Optics 2 (1997), , 364-74). Ratios outside the 95% confidence interval were determined to be significantly changed by the treatment.

Table 1. List of 93 genes on the array for determination of non-specific effect function for siRNA cell transfection effects. The table is organized in 3 pathways (apoptosis, IFN response, DNA repair/synthesis)

| N° | Gene symbol | Gene name | Pathways |
|---|---|---|---|
| 1 | BAD | BCL2-antagonist of cell death | Apoptosis |
| 2 | BAX | BCL2-associated X protein | Apoptosis |
| 3 | BCL2 | B-cell lymphoma2 | Apoptosis |
| 4 | BCLX | BCL2-like1/BCLX | Apoptosis |
| 5 | BID | BH3 interacting domain death agonist | Apoptosis |
| 6 | CASP2 | Caspase2 | Apoptosis |
| 7 | CASP3 | Caspase3 | Apoptosis |
| 8 | CASP7 | Caspase7 | Apoptosis |
| 9 | CASP8 | Caspase8 | Apoptosis |
| 10 | CASP9 | Caspase9 | Apoptosis |
| 11 | ABL1 | v-abl Abelson murine leukemia viral oncogene homolog 1 | Apoptosis |
| 12 | ADAM17 | a disintegrin and metalloproteinase domain 17 (tumor necrosis factor, alpha, converting | Apoptosis |
| 13 | CASP1 | caspase 1, apoptosis-related cysteine protease (interleukin 1, beta, convertase) | Apoptosis |
| 14 | CASP10 | Caspase 10, apoptosis-related cysteine protease | Apoptosis |
| 15 | CYCS | Cytochrome c, somatic | Apoptosis |
| 16 | E2F1 | E2F transcription factor 1 | Apoptosis |
| 17 | FADD | Fas (TNFRSF6)-associated via death domain | Apoptosis |
| 18 | FASN | Fatty acid synthase | Apoptosis |
| 19 | GSTT1 | glutathione S-transferase theta 1 | Apoptosis |
| 20 | IGF2 | insulin-like growth factor 2 (somatomedin A) | Apoptosis |
| 21 | IGFBP4 | insulin-like growth factor binding protein 4 | Apoptosis |
| 22 | JUN | v-jun sarcoma virus 17 oncogene homolog (avian) | Apoptosis |
| 23 | LTA | lymphotoxin alpha (TNF superfamily, member 1) | Apoptosis |
| 24 | MAPK1 | mitogen-activated protein kinase 1 | Apoptosis |
| 25 | MAPK3 | mitogen-activatea protein Kinase 3 | Apoptosis |
| 26 | B2M | Beta-2-microglobulin | IFN response |
| 27 | C2 | Complement component 2 | IFN response |
| 28 | CNP | 2',3'-cyclic nudeotide 3' phosphodiesterase | IFN response |
| 29 | DUSP1 | Dual specificity phosphatase 1 | IFN response |
| 30 | ENO1 | Enolase 1, (alpha) | IFN response |
| 31 | ENPP1 | Ectonucleotide pyrophosphatase/ phosphodiesterase 1 | IFN response |

Table continued

| N° | Gene symbol | Gene name | Pathways |
|---|---|---|---|
| 32 | FCER1G | Fc fragment of IgE, high affinity I, receptor for; gamma polypeptide | IFN response |
| 33 | G1P3 | interferon, alpha-inducible protein (clone IFI-6-16) | IFN response |
| 34 | GEM | GTP binding protein overexpressed in skeletal muscle | IFN response |
| 35 | HLA-C | Major histocompatibility complex, class I, C | IFN response |
| 36 | IFIT2 | Interferon-induced protein with tetratricopeptide repeats 2 | IFN response |
| 37 | IFITM1 | Interferon induced transmembrane protein 1 (9-27) | IFN response |
| 38 | IFITM2 | Interferon induced transmembrane protein 2 (1-8D) | IFN response |
| 39 | IFNAR1 | Interferon (alpha, beta and omega) receptor 1 | IFN response |
| 40 | IRF1 | Interferon regulatory factor 1 | IFN response |
| 41 | JAK1 | Janus kinase 1 (a protein tyrosine kinase) | IFN response |
| 42 | JAK2 | Janus kinase 2 (a protein tyrosine kinase) | IFN response |
| 43 | KLK3 | Kallikrein 3, (prostate specific antigen) | IFN response |
| 44 | MET | Met proto-oncogene (hepatocyte growth factor receptor) | IFN response |
| 45 | MX1 | Myxovirus (influenza virus) resistance 1, interferon-inducible protein p78 (mouse) | IFN response |
| 46 | MYC | V-myc myelocytomatosis viral oncogene homolog (avian) | IFN response |
| 47 | NRG1 | Neuregulin 1 | IFN response |
| 48 | NYREN18 | NEDD8 ultimate buster-1 | IFN response |
| 49 | OAS1 | 2',5'-oligoadenylate synthetase 1, 40/46kDa | IFN response |
| 50 | OAS2 | 2'-5'-oligoadenylate synthetase 2, 69/71kDa | IFN response |
| 51 | OAS3 | 2'-5'-oligoadenylate synthetase 3, 100kDa | IFN response |
| 52 | PLA2G1B | Phospholipase A2, group IB (pancreas) | IFN response |
| 53 | PML | Promyelocytic leukemia | IFN response |
| 54 | PRAME | Preferentially expressed antigen in melanoma | IFN response |
| 55 | SH2D1A | SH2 domain protein 1A, Duncan's disease (lymphoproliferative syndrome) | IFN response |
| 56 | SLC1A2 | Solute carrier family 1 (glial high affinity glutamate transporter), member 2 | IFN response |
| 57 | SPP1 | Secreted phosphoprotein 1 | IFN response |
| 58 | STAT1 | Signal transducer and activator of transcription 1, 91 kDa | IFN response |
| 59 | TAP1 | Transporter 1, ATP-binding cassette, sub-family B (MDR/TAP) | IFN response |
| 60 | TNFSF10 | Tumor necrosis factor (ligand) superfamily, member 10 | IFN response |
| 61 | UBE2L6 | Ubiquitin-conjugating enzyme E2L 6 | IFN response |
| 62 | WARS | Tryptopnanyl-tRNA synthetase | IFN response |

Table continued

| N° | Gene symbol | Gene name | Pathways |
|---|---|---|---|
| 63 | ADPRT | ADP-ribosyitransferase | DNA |
| 64 | CROC1A | Ubiquitin-conjugating enzyme E2 variant1 | DNA |
| 65 | FHIT | Fragile histidine triad gene | DNA |
| 66 | GADD153 | DNA-damage-inducible transcript3 | DNA |
| 67 | PLK | Polo-like kinase | DNA |
| 68 | POLA2 | Polymerase alpha | DNA |
| 69 | RRM1 | Ribonucleotide-reductase M1 polypeptide | DNA |
| 70 | TERT | Telomerase-reverse transcriptase | DNA |
| 71 | TOP2 | Topoisomerase2-alpha | DNA |
| 72 | TRF1 | Telomeric repeat binding factor1 | DNA |
| 73 | TYMS | Thymidylate-synthetase | DNA |
| 74 | BCL6 | B-cell CLL/lymphoma 6 (zinc finger protein 51) | DNA |
| 75 | BRCA1 | Breast cancer 1, early onset | DNA |
| 76 | BRCA2 | Breast cancer 2, early onset | DNA |
| 77 | ERCC1 | Excision repair cross-complementing rodent repair deficiency, complementation group 1 | DNA |
| 78 | ERCC2 | Excision repair cross-complementing rodent repair deficiency, complementation group 2 | DNA |
| 79 | ERCC3 | Excision repair cross-complementing rodent repair deficiency, complementation group 3 | DNA |
| 80 | FEN1 | Flap structure-specific endonuclease 1 | DNA |
| 81 | GADD45A | growth arrest and DNA-damage-inducible, alpha | DNA |
| 82 | MLH1 | MutL homolog 1, colon cancer, nonpolyposis type 2 (E. coli) | DNA |
| 83 | MSH2 | MutS homolog 2, colon cancer, nonpolyposis type 1 (E. coli) | DNA |
| 84 | PRKDC | Protein kinase, DNA-activated, catalytic polypeptide | DNA |
| 85 | RAD50 | RAD50 homolog (S. cerevisiae) | DNA |
| 86 | RAD51 | RAD51 homolog (RecA homolog, E. coli) (S. cerevisiae) | DNA |
| 87 | RAD52 | RAD52 homolog (S. cerevislae) | DNA |
| 88 | TNFRSF10B | Tumor necrosis factor receptor superfamily, member 10b | DNA |
| 89 | TNFRSF4 | Tumor necrosis factor receptor superfamily, member 4 | DNA |
| 90 | TNFRSF6 | Tumor necrosis factor receptor superfamily, member 6 | DNA |
| 91 | UNG | Uracil-DNA glycosylase | DNA |
| 92 | XPA | Xeroderma pigmentosum, complementation group A | DNA |
| 93 | XRCC1 | X-ray repair complementing defective repair in Chinese hamster cells 1 | DNA |

Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping

| N* | Gene symbol | Gene name | Pathways |
|---|---|---|---|
| 1 | ABL1 | v-abl Abelson munne leukemia viral oncogene homolog 1 | Apoptosis |
| 2 | ADAM17 | a disintegrin and metalloproteinase domain 17 (tumor necrosis factor, alpha, converting enzym | Apoptosis |
| 3 | BAD | BCL2-antagonist of cell death | Apoptosis |
| 4 | BAX | BCL2-associated X protein | Apoptosis |
| 5 | BCL2 | B-cell lymphoma2 | Apoptosis |
| 6 | BCLX | BCL2-like1/BCLX | Apoptosis |
| 7 | BID | BH3 interacting domain death agonist | Apoptosis |
| 8 | CASP1 | caspase 1, apoptosis-related cysteine protease (interleukin 1, beta, convertase) | Apoptosis |
| 9 | CASP10 | Caspase 10, apoptosis-related cysteine protease | Apoptosis |
| 10 | CASP2 | Caspase2 | Apoptosis |
| 11 | CYCS | Cytochrome c, somatic | Apoptosis |
| 12 | E2F1 | E2F transcription factor 1 | Apoptosis |
| 13 | FADD | Fas (TNFRSF6)-associated via death domain | Apoptosis |
| 14 | FASN | Fatty acid synthase | Apoptosis |
| 15 | GSTT1 | glutathione S-transferase theta 1 | Apoptosis |
| 16 | IGF2 | insulin-like growth factor 2 (somatomedin A) | Apoptosis |
| 17 | IGFBP4 | insulin-like growth factor binding protein 4 | Apoptosis |
| 18 | JUN | v-jun sarcoma virus 17 oncogene homolog (avian) | Apoptosis |
| 19 | LTA | lymphotoxin alpha (TNF superfamily, member 1) | Apoptosis |
| 20 | MAPK1 | mitogen-activated protein kinase 1 | Apoptosis |
| 21 | MAPK3 | mitogen-activated protein kinase 3 | Apoptosis |
| 22 | CASP3 | Caspase3 | Apoptosis |
| 23 | CASP7 | Caspase7 | Apoptosis |
| 24 | CASP8 | Caspase8 | Apoptosis |
| 25 | CASP9 | Caspase9 | Apoptosis |
| 26 | CATB | Catenin , beta 1 | Cell adhesion |
| 27 | CDH1 | Cadherine1, type 1,E-cadherine | Cell adhesion |
| 28 | CDH11 | Cadherine11, type 2, OB-cadherin | Cell adhesion |
| 29 | CDH13 | Cadherine13, H-cadherin | Cell adhesion |

Table continued

Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping

| N* | Gene symbol | Gene name | Pathways |
|---|---|---|---|
| 30 | ICAM-1 | Intracellular adhesion molecule1 | Cell adhesion |
| 31 | ITGA5 | Integrin alpha5 | Cell adhesion |
| 32 | ITGA6 | Integrin alpha6 | Cell adhesion |
| 33 | ITGB1 | Integrin beta1 | Cell adhesion |
| 34 | TSP1 | Thrombospondin 1 | Cell adhesion |
| 35 | TSP2 | Thrombospondin 2 | Cell adhesion |
| 36 | ATM | Ataxia telangiectasia mutated | Cell cycle |
| 37 | CAV1 | Caveolin1 | Cell cycle |
| 38 | CCNA1 | CyclinA1 | Cell cycle |
| 39 | CCNB1 | CyclinB1 | Cell cycle |
| 40 | CCND1 | CyclinD1 | Cell cycle |
| 41 | CCND2 | CydinD2 | Cell cycle |
| 42 | CCND3 | CydinD3 | Cell cycle |
| 43 | CCNE1 | CyclinE1 | Cell cycle |
| 44 | CCNF | CydinF | Cell cycle |
| 45 | CCNH | CyclinH | Cell cycle |
| 46 | CDK2 | Cyclin-dependent kinase2 | Cell cycle |
| 47 | CDK4 | Cyclin-dependent kinase4 | Cell cycle |
| 48 | CDK6 | Cyclin-dependent kinase6 | Cell cycle |
| 49 | DHFR | Dihydrofolate reductase | Cell cycle |
| 50 | GRB2 | Growth factor receptor-bound protein2 | Cell cycle |
| 51 | MKI67 | antigen identified by monoclonal antibody Ki-67 | Cell cycle |
| 52 | MDM2 | MDM2 | Cell cycle |
| 53 | p16 | Cyclin dependent kinase inhibitor 2A | Cell cycle |
| 54 | p21 | Cyclin dependent kinase inhibitor 1A | Cell cycle |
| 55 | p27 | Cyclin dependent kinase inhibitor 1B | Cell cycle |
| 56 | p35 | Cyclin dependent kinase5 regulatory subunit1 | Cell cycle |
| 57 | p53 | Tumor protein p53 | Cell cycle |
| 58 | p57 | Cyclin dependent kinase inhibitor 1C | Cell cycle |
| 59 | PCNA | Proliferating cell nuclear antigen | Cell cycle |
| 60 | RB1 | Retinoblastome1 | Cell cycle |
| 61 | S100A4 | S100 calcium binding protein A4 | Cell cycle |
| 62 | S100A8 | Calprotectin / S100 calcium binding protein A8 (calgranulin A) | Cell cycle |
| 63 | SMAD1 | MAD,mothers against decapentaplegic homolog 1 | Cell cycle |

Table continued

Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping

| N* | Gene symbol | Gene name | Pathways |
|---|---|---|---|
| 64 | SMAD2 | MAD,mothers against decapentaplegic homolog 2 | Cell cycle |
| 65 | SMAD3 | MAD,mothers against decapentaplegic homolog 3 | Cell cycle |
| 66 | SMAD4 | MAD,mothers against decapentaplegic homolog 4 | Cell cycle |
| 67 | TK1 | Thymidine-kinase 1 | Cell cycle |
| 68 | SPRR1B B | Cornifin/ small proline-rich protein 1B | Cell differentiation |
| 69 | CSF1R | Colony stimulating factor 1 receptor | Cell signaling / receptor |
| 70 | EGFR | Epidermal growth factor receptor | Cell signaling / receptor |
| 71 | ESR2 | Estrogen receptor 2 | Cell signaling / receptor |
| 72 | FGFR | Fibroblast growth factor receptor 1 | Cell signaling / receptor |
| 73 | IGF1R | Insulin like growth factor1 receptor | Cell signaling / receptor |
| 74 | IL11RA | Interleukin 11-receptor-alpha | Cell signaling / receptor |
| 75 | NCK1 | NCK adaptor protein1 | Cell signaling / receptor |
| 76 | NCOR1 | Nuclear receptor co-repressor 1 | Cell signaling / receptor |
| 77 | NCOR2 | Nuclear receptor co-repressor 2 | Cell signaling / receptor |
| 78 | PGR | Progesterone receptor | Cell signaling / receptor |
| 79 | TBXA2R | Thromboxane-A2-receptor | Cell signaling / receptor |
| 80 | PLAUR | Plasminogen activator, Urokinase-receptor | Cell signaling / receptor |
| 81 | VEGFR1 | Vascular endothelial growth factor receptor1/ fms-related tyrosine kinase 1 | Cell signaling / receptor |
| 82 | VEGFR2 | Vascular endothelial growth factor receptor2 / Kinase insert domain receptor | Cell signaling / receptor |
| 83 | VEGFR3 | Vascular endothelial growth factor receptor3/ fms-related tyrosine kinase 4 | Cell signaling / receptor |
| 84 | CDC42 | Cell division cycle42 | Cell structure |
| 85 | EMS1 | Ems1 sequence | Cell structure |
| 86 | GSN | Gelsolin | Cell structure |
| 87 | ON | Osteonectin, secreted protein, acidic, cysteine-rich | Cell structure |
| 88 | PAK | P21 activated kinase1 | Cell structure |
| 89 | SM22 | Transgelin | Cell structure |
| 90 | TB10 | Thymosin beta 10 | Cell structure |
| 91 | CENPF | Mitesin, centromere protein F | Chromosomal processing |

Table continued

Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping

| N* | Gene symbol | Gene name | Pathways |
|---|---|---|---|
| 92 | H2B/S | histone2b member B/S consensus/ Histone 1, H2bk | Chromosomal processing |
| 93 | H3FF | HIstone3 member F consensus/ Histone 1, H31 | Chromosomal processing |
| 94 | H4FM | Histone4 member M consensus/ Histone 1,H41 | Chromosomal processing |
| 95 | KNSL5 | Mitotic-kinesin-like-protein1 / kinesin family member 23 | Chromosomal processing |
| 96 | KNSL6 | Mitotic-centromere-associated-kinesin Kinesin family member 2C | Chromosomal processing |
| 97 | PAI1 | Plasminogen activator inhibitor type1/ serine proteinase inhibitor. clade E,member 1 | Circulation |
| 98 | PAI2 | Plasminogen activator inhibitor type2/ serine proteinase inhibitor,clade B,member 2 | Circulation |
| 99 | PLAU | Plasminogen activator, Urokinase | Circulation |
| 100 | TPA | Plasminogen activator tissue | Circulation |
| 101 | VWF | von willebrand Factor | Circulation |
| 102 | BCL6 | B-cell CLL/lymphoma 6 (zinc finger protein 51) | DNA repair/synthesis |
| 103 | BRCA1 | Breast cancer 1, early onset | DNA repair/synthesis |
| 104 | BRCA2 | Breast cancer 2, early onset | DNA repair/synthesis |
| 105 | ERCC1 | Excision repair cross-complementing rodent repair deficiency, complementation group 1 | DNA repair/synthesis |
| 106 | ERCC2 | Excision repair cross-complementing rodent repair deficiency, complementation group 2 | DNA repair/synthesis |
| 107 | ERCC3 | Excision repair cross-complementing rodent repair deficiency, complementation group 3 | DNA repair/synthesis |
| 108 | FEN1 | Flap structure-specific endonuclease 1 | DNA repair/synthesis |
| 109 | GADD45A | growth arrest and DNA-damage-inducible, alpha | DNA repair/synthesis |
| 110 | MLH1 | MutL homolog 1, colon cancer, nonpolyposis type 2 (E. coli) | DNA repair/synthesis |
| 111 | MSH2 | MutS homolog 2, colon cancer, nonpolyposis type 1 (E. coli) | DNA repair/synthesis |

Table continued

| Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping | | | |
|---|---|---|---|
| N* | Gene symbol | Gene name | Pathways |
| 112 | PRKDC | Protein kinase, DNA-activated, catalytic polypeptide | DNA repair/synthesis |
| 113 | RAD50 | RAD50 homolog (S. cerevisiae) | DNA repair/synthesis |
| 114 | RAD51 | RAD51 homolog (RecA homolog, E. coli) (S. cerevisiae) | DNA repair/synthesis |
| 115 | RAD52 | RAD52 homolog (S. cerevisiae) | DNA repair/synthesis |
| 116 | TNFRSF10B | Tumor necrosis factor receptor superfamily, member 10b | DNA repair/synthesis |
| 117 | TNFRSF4 | Tumor necrosis factor receptor superfamily, member 4 | DNA repair/synthesis |
| 118 | TNFRSF6 | Tumor necrosis factor receptor superfamily, member 6 | DNA repair/synthesis |
| 119 | UNG | Uracil-DNA glycosylase | DNA repair/synthesis |
| 120 | XPA | Xeroderma pigmentosum, complementation group A | DNA repair/synthesis |
| 121 | XRCC1 | X-ray repair complementing defective repair in Chinese hamster cells 1 | DNA repair/synthesis |
| 122 | ADPRT | ADP-ribosyltransferase | DNA repair/synthesis |
| 123 | CROC1A | Ubiquitin-conjugating enzyme E2 variant1 | DNA repair/synthesis |
| 124 | FHIT | Fragile histidine triad gene | DNA repair/synthesis |
| 125 | GADD153 | DNA-damage-inducible transcript3 | DNA repair/synthesis |
| 126 | PLK | Polo-like kinase | DNA repair/synthesis |
| 127 | POLA2 | Polymerase alpha | DNA repair/synthesis |
| 128 | RRM1 | Ribonucleotide-reductase M1 polypeptide | DNA repair/synthesis |
| 129 | TERT | Telomerase-reverse transcriptase | DNA repair/synthesis |
| 130 | TOP2 | Topoisomerase2-alpha | DNA repair/synthesis |
| 131 | TRF1 | Telomeric repeat binding factor1 | DNA repair/synthesis |
| 132 | TYMS | Thymidylate-synthetase | DNA repair/synthesis |
| 133 | BSG | Basigin | Extracellular matrix |
| 134 | COL6A2 | Collagen, type VI, alpha 2 | Extracellular matrix |
| 135 | FN1 | fibronectin 1 | Extracellular matrix |
| 136 | MMP1 | Matrix metalloproteinase 1 | Extracellular matrix |
| 137 | MMP11 | Matrix metalloproteinase 11 | Extracellular matrix |
| 138 | MMP12 | Matrix metalloproteinase 12 | Extracellular matrix |
| 139 | MMP13 | Matrix metalloproteinase 13 | Extracellular matrix |
| 140 | MMP14 | Matrix metalloproteinase 14 | Extracellular matrix |
| 141 | MMP15 | Matrix metalloproteinase 15 | Extracellular matrix |
| 142 | MMP2 | Matrix metalloproteinase 2 | Extracellular matrix |
| 143 | MMP3 | Matrix metalloproteinase 3 | Extracellular matrix |

Not applicable

Table continued

| Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping | | | |
|---|---|---|---|
| N* | Gene symbol | Gene name | Pathways |
| 144 | MMP7 | Matrix metalloproteinase 7 | Extracellular matrix |
| 145 | MMP9 | Matrix metalloproteinase 9 | Extracellular matrix |
| 146 | OPN | Osteopontin/ secreted phosphoprotein 1 | Extracellular matrix |
| 147 | TIMP1 | Tissue inhibitor of metalloproteinase1 | Extracellular matrix |
| 148 | TIMP2 | Tissue inhibitor of metalloproteinase2 | Extracellular matrix |
| 149 | BMP2 | Bone morphogenetic protein2 | Growth factors and cytokines |
| 150 | CSF1 | Colony stimulating factor 1 | Growth factors and cytokines |
| 151 | CTGF | Connective tissue growth factor | Growth factors and cytokines |
| 152 | FGF2 | Fibroblast growth factor 2 | Growth factors and cytokines |
| 153 | FGF8 | Fibroblast growth factor 8 | Growth factors and cytokines |
| 154 | IGF1 | Insulin-like growth factor1 | Growth factors and cytokines |
| 155 | IGFBP2 | Insulin-like growth factor binding protein2 | Growth fadors and cytokines |
| 156 | IGFBP3 | Insulin-like growth factor binding protein3 | Growth factors and cytokines |
| 157 | IGFBP5 | Insulin-like growth factor binding proteins | Growth factors and cytokines |
| 158 | IL10 | Interleukin 10 | Growth factors and cytokines |
| 159 | IL11 | Interleukin 11 | Growth factors and cytokines |
| 160 | IL15 | Interleukin 15 | Growth factors and cytokines |
| 161 | IL1A | Interleukin1 alpha | Growth factors and cytokines |
| 162 | IL1B | Interleukin1 beta | Growth factors and cytokines |
| 163 | IL4 | Interleukin 4 | Growth factors and cytokines |
| 164 | IL6 | Interleukin 6 | Growth factors and cytokines |
| 165 | IL8 | Interleukin 8 | Growth fadors and cytokines |
| 166 | MEK1 | Mitogen activated protein kinase kinase1 | Growth factors and cytokines |
| 167 | MEK2 | Mitogen activated protein kinase kinase2 | Growth factors and cytokines |
| 168 | TNFA | Tumor necrosis factor | Growth factors and cytokines |
| 169 | VEGF | Vascular endothelial growth factor | Growth factors and cytokines |
| 170 | VEGFB | Vascular endothelial growth factor B | Growth factors and cytokines |
| 171 | VEGFC | Vascular endothelial growth factor C | Growth factors and cytokines |
| 172 | VEGFD | Vascular endothelial growth factor D/ c-fos induced growth factor | Growth factors and cytokines |
| 173 | B2M | Beta-2-microglobulin | IFN response |
| 174 | C2 | Complement component 2 | IFN response |

Table continued

Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping

| N* | Gene symbol | Gene name | Pathways |
|---|---|---|---|
| 175 | CNP | 2',3'-cyclic nucleotide 3' phosphodiesterase | IFN response |
| 176 | DUSP1 | Dual specificity phosphatase 1 | IFN response |
| 177 | ENO1 | Enolase 1, (alpha) | IFN response |
| 178 | ENPP1 | Edonudeotide pyrophosphatase/ phosphodiesterase 1 | IFN response |
| 179 | FCER1G | Fc fragment of IgE, high affinity I, receptor for, gamma polypeptide | IFN response |
| 180 | G1P3 | Interferon, alpha-inducible protein (done IFI-6-16) | IFN response |
| 181 | GEM | GTP binding protein overexpressed in skeletal muscle | IFN response |
| 182 | HLA-C | Major histocompatibility complex, class I, C | IFN response |
| 183 | IFIT2 | Interferon-induced protein with tetratricopeptide repeats 2 | IFN response |
| 184 | IFITM1 | Interferon induced transmembrane protein 1 (9-27) | IFN response |
| 185 | IFITM2 | Interferon induced transmembrane protein 2 (1-8D) | IFN response |
| 186 | IFNAR1 | Interferon (alpha, beta and omega) receptor 1 | IFN response |
| 187 | IRF1 | Interferon regulatory factor 1 | IFN response |
| 188 | JAK1 | Janus kinase 1 (a protein tyrosine kinase) | IFN response |
| 189 | JAK2 | Janus kinase 2 (a protein tyrosine kinase) | IFN response |
| 190 | KLK3 | Kallikrein 3, (prostate specific antigen) | IFN response |
| 191 | MET | Met proto-oncogene (hepatocyte growth factor receptor) | IFN response |
| 192 | MX1 | Myxovirus (influenza virus) resistance 1, interferon-inducible protein p78 (mouse) | IFN response |
| 193 | MYC | V-myc myelocytomatosis viral oncogene homolog (avian) | IFN response |
| 194 | NRG1 | Neuregulin 1 | IFN response |
| 195 | NYREN18 | NEDD8 ultimate buster-1 | IFN response |
| 196 | OAS1 | 2',5'-oligoadenylate synthetase 1, 40/46kDa | IFN response |
| 197 | OAS2 | 2'-5'-oligoadenylate synthetase 2, 69/71kDa | IFN response |

Table continued

Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping

| N* | Gene symbol | Gene name | Pathways |
|---|---|---|---|
| 198 | OAS3 | 2'-5'-oligoadenylate synthetase 3, 100kDa | IFN response |
| 199 | PLA2G1B | Phospholipase A2, group IB (pancreas) | IFN response |
| 200 | PML | Promyelocytic leukemia | IFN response |
| 201 | PRAME | Preferentially expressed antigen in melanoma | IFN response |
| 202 | SH2D1A | SH2 domain protein 1A, Duncan's disease (lymphoproliterative syndrome) | IFN response |
| 203 | SLC1A2 | Solute carrier family 1 (glial high affinity glutamate transporter), member 2 | IFN response |
| 204 | SPP1 | Secreted phosphoprotein 1 | IFN response |
| 205 | STAT1 | Signal transducer and activator of transcription 1, 91 kDa | IFN response |
| 208 | TAP1 | Transporter 1, ATP-binding cassette, sub-family B (MDR/TAP) | IFN response |
| 207 | TNFSF10 | Tumor necrosis factor (ligand) superfamily, member 10 | IFN response |
| 208 | UBE2L6 | Ubiquitin-conjugating enzyme E2L 6 | IFN response |
| 209 | WARS | Tryptophanyl-tRNA synthetase | IFN response |
| 210 | CKB | Creatin-kinase, brain | Intermediate metabolism |
| 211 | ETFB | Electron-transfer-flavoprotein, beta polypeptide | Intermediate metabolism |
| 212 | G6PD | Glucose-6-phosphate dehydrogenase | Intermediate metabolism |
| 213 | GLB1 | Beta1-galactosidase | Intermediate metabolism |
| 214 | ODC | Omithine decarboxylase1 | Intermediate metabolism |
| 215 | PKM2 | Pyruvate-kinase-muscle | Intermediate metabolism |
| 216 | ANX1 | Annexin A1 | Lipid metabolism |
| 217 | APOJ | Clusterin / ApoliproteinJ | Lipid metabolism |
| 218 | COX2 | Prostaglandin-endoperoxidase synthase 2 | Lipid metabolism |
| 219 | c-myc | c-myc binding protein | Oncogenesis |
| 220 | FES | Feline sarcoma oncogene | Oncogenesis |
| 221 | FOS | c-fos/v-fos FBJ murine osteosarcoma viral oncogene homolog | Oncogenesis |
| 222 | RAF1 | c-raf-1 / v-raf-1 murine leukemia viral oncogene homolog 1 | Oncogenesis |
| 223 | SHC | SHC transforming protein1 | Oncogenesis |

Table continued

| Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping | | | |
|---|---|---|---|
| N* | Gene symbol | Gene name | Pathways |
| 224 | GPX | Glutathione peroxidase 1 | Oxidative metabolism |
| 225 | GSTP1 | Glutathione S-transferase pi | Oxidative metabolism |
| 226 | MSRA | Methionine-sulfoxide-redudase A/peptide | Oxidative metabolism |
| 227 | $SOD_2$ | Superoxide dismutase2 | Oxidative metabolism |
| 228 | PSMD11 | 26S-proteasome-subunit-p44.5 | Proteasome |
| 229 | UBE2C | Ubiquitin conjugating enzyme E2C/ubiquitin carrier protein | Proteasome |
| 230 | ADAM1 | A disintegrin and metalloproteinase domain 1 | Protein metabolism |
| 231 | CANX | Calnexin | Protein metabolism |
| 232 | CTSB | CathepsinB | Protein metabolism |
| 233 | CTSD | CathepsinD | Protein metabolism |
| 234 | CTSL | CathepsinL | Protein metabolism |
| 235 | EIF4-E | Eukaryotic translation initiation factor 4E | Protein metabolism |
| 236 | AOP2 | Anti-oxidant-protein2 | Stress response |
| 237 | HMOX | Heme oxygenase 1 | Stress response |
| 238 | HSP27 | Heat shock 27kDa protein1 | Stress response |
| 239 | HSP40 | Heat shock 40kD protein1/ DnaJ (Hsp40)homolog | Stress response |
| 240 | HSP70 | Heat shock 70kDa protein4 | Stress response |
| 241 | HSP90-a | Heat shock 90kDa protein1,alpha | Stress response |
| 242 | HSP90-b | Heat shock 90kDa protein 1, beta | Stress response |
| 243 | JNK1 | Mitogen activated protein kinase8 | Stress response |
| 244 | JNK2 | Mitogen activated protein kinase9 | Stress response |
| 245 | JNK3 | Mitogen-activated protein kinase 10 | Stress response |
| 246 | DP1 | Transcription factor Dp-1 | Transcription |
| 247 | DP2 | transcription factor Dp-2 | Transcription |
| 248 | E2F2 | E2F transcription factor2 | Transcription |
| 249 | E2F3 | E2F transcription factor3 | Transcription |
| 250 | E2F4 | E2F transcription factor4 | Transcription |
| 251 | EGR1 | Early growth response1 | Transcription |
| 252 | EGR3 | Early growth response3 | Transcription |
| 253 | JUND | Jun D proto-oncogene | Transcription |
| 254 | MAX | MAX protein | Transcription |
| 255 | MYBL2 | v-myb myeloblastosis viral oncogene homolog-like2/ b-myb | Transcription |
| 256 | TFAP2A | Transcription factor AP-2 alpha | Transcription |

Table continued

| Table 2. List of 274 genes on the array classified according to their non specific target functions (apoptosis, DNA repair/synthesis, IFN response) and vital functions (cell adhesion, cell cycle, cell signaling/receptor, cell structure, chromosomal processing, growth factors/cytokines, intermediate metabolism, extracellular matrix, oxidative metabolism, protein metabolism, transcription). The table also provides some genes which belong to other categories (cell differentiation, circulation, lipid metabolism, oncogenesis, tumor supressor, stress response, proteasome, housekeeping | | | |
|---|---|---|---|
| N* | Gene symbol | Gene name | Pathways |
| 257 | TFAP2B | Transcription factor AP-2 beta | Transcription |
| 258 | TFAP2C | Transcription factor AP-2 gamma | Transcription |
| 259 | BIN1 | Bridging integrator 1 | Tumor suppressor |
| 260 | ING1 | Inhibitor of growth family, member 1 | Tumor suppressor |
| 261 | TGFBR2 | TGF-beta-R2 | Tumor suppressor |
| 262 | RPL13A | 23KDa Highly basic protein | Housekeeping gene |
| 263 | ALDOA | Aldolase A, fructose biphosphate | Housekeeping gene |
| 264 | K-ALPHA-1 | tubulin, alpha, ubiquitous | Housekeeping gene |
| 265 | ACTB | Actin, beta | Housekeeping gene |
| 266 | PPIE | Cyclophilin 33 | Housekeeping gene |
| 267 | GAPD | Glyceraldehyde-3-phosphate-dehydrogenase | Housekeeping gene |
| 268 | HK1 | Hexokinase 1 | Housekeeping gene |
| 269 | HPRT1 | Hypoxanthine phosphoribosyltransferase 1 | Housekeeping gene |
| 270 | MDH1 | Malate dehydrogenase 1, NAD (soluble) | Housekeeping gene |
| 271 | YWHAZ | Phospholipase A2 | Housekeeping gene |
| 272 | RPS9 | Ribosomal protein S9 | Housekeeping gene |
| 273 | SDS | Serine dehydratase | Housekeeping gene |
| 274 | TFRC | Transferrin receptor | Housekeeping gene |

**Claims**

1. A method for determining and/or quantifying the effects of a siRNA transfection in cell(s), said method comprising the steps of:

   providing an array, having fixed on a substrate a maximum of 2999 different capture molecules specific of nucleic acids or proteins belonging to or representative for each of the following 3 non-specific effect functions: apoptosis, DNA repair/synthesis and
   interferon response wherein the array allows the detection of at least 20 different said nucleic acids or proteins, contacting a sample component derived from said transfected cell(s) with the array,
   detecting and/or quantifying the intensity of the signal on said capture molecules,
   comparing the transcriptome or proteome of said transfected cell(s) with at least one reference or control condition,
   wherein the pattern and/or the intensity of the binding events is indicative of the potentially deleterious non-specific side effects of the presence of siRNA in said transfected cell(s).

2. A method for determining and/or quantifying the effects of a siRNA transfection in cell(s), said method comprising the steps of:

   providing an array, having fixed on a substrate a maximum of 2999 different capture molecules specific of nucleic acids or proteins belonging to or representative of each of the following 3 non-specific effect functions: apoptosis, DNA repair/synthesis and interferon response and at least one nucleic acid or protein, belonging to or representative for at least 4 of the following vital cellular functions: cell adhesion, cell cycle, growth factors and cytokines, cell signaling/receptor, chromosomal processing, intermediate metabolism, extracellular matrix, cell structure, protein metabolism, oxidative metabolism, transcription and housekeeping genes wherein the array allows the detection of at least 20 different said nucleic acids or proteins,

contacting a sample component derived from said transfected cell(s) with the array,

detecting and/or quantifying the intensity of the signal on said capture molecules,

comparing the transcriptome or proteome of said transfected cell(s) with at least one reference or control condition,

wherein the pattern and/or the intensity of the binding events is indicative of the consequence of inhibition of a particular gene expression due to the presence of a siRNA on cellular vital functions and its potentially deleterious non-specific side effects of the presence of siRNA in said transfected cell(s).

3. A method for screening for an agent capable of modulating the effect of a siRNA, comprising the steps of:

providing an array, having fixed on a substrate a maximum of 2999 different capture molecules specific of nucleic acids or proteins belonging to or representative of each of the following 3 non-specific effect functions: apoptosis, DNA repair/synthesis and interferon response and at least one nucleic acid or protein, belonging to or representative for at least 4 of the following vital cellular functions: cell adhesion, cell cycle, growth factors and cytokines, cell signaling/receptor, chromosomal processing, intermediate metabolism, extracellular matrix, cell structure, protein metabolism, oxidative metabolism, transcription and housekeeping genes wherein the array allows the detection of at least 20 different said nucleic acids or proteins,

exposing cells transfected with siRNA with an agent,

contacting a sample component derived from said transfected cell(s) with the array,

detecting the expression level of at least one gene associated with each of at least 2 of the 3 non-specific effect functions and at least 10 other genes associated with at least 3 vital functions as listed in table 2.

comparing the expression level of the transfected cell(s) exposed to the agent with at least one reference or control condition comprising the transcriptome or proteome of transfected cells that had not been exposed to the agent,

wherein the pattern and/or the intensity of the binding events is indicative of the effect of the agent on the inhibition of a particular gene expression due to the presence of a siRNA on cellular vital functions.

4. The method according to any of claims 1 - 3,
wherein the array also comprises capture molecules for the detection of the gene targeted by the transfected siRNA; or
wherein the array comprises more than 50 different capture molecules and less than 1000; or
wherein the array comprises capture molecules for the detection of at least 20 and preferably 50 and still preferably 100 genes or all the genes according to the list provided in table 2; or
wherein the array comprises at least 20 and preferentially more than 50 different capture molecule with at least one capture molecule for each of the 3 non-specific effect functions; or
wherein the array comprises at least 20 and preferentially more than 50 different capture molecules with at least 5 different capture molecule for each of the 3 non-specific effect functions; or
wherein the array comprises at least 5 different capture molecule for each of the 3 non-specific effect functions and at least 5 different capture molecule of at least 4 vital cellular functions; or
wherein the array comprises at least 5 different capture molecule for each of the 3 non-specific effect functions and at least 20 different capture molecule of at least 4 vital cellular functions; or
wherein the array comprises capture molecules being polynucleotides present on a solid substrate; or
wherein the array comprises antibodies or related proteins having specific affinity for proteins.

5. The method according to any of claims 1 - 3,
wherein the step of detecting and optionally quantifying the intensity of the signal is performed on a single capture nucleotide species; or
wherein the values for the quantification on the array are taken as the average of three experimental data.

6. The method according to any of claims 1 - 3,
wherein the 3 non-specific effect functions on the array are represented by at least 1 gene per function from table 1; or
wherein the 3 non-specific effect functions on the array are represented by at least 5 genes per function from table 1.

**7.** The method according to any of claims 1 - 3,
wherein the cellular vital functions on the array are represented by at least 5 genes from table 2; or.
wherein the cellular vital functions on the array are represented by at least 20 genes from table 2; or
wherein at least one gene for each of the 11 vital functions is a gene which effects a regulatory activity in the function.

**8.** The method according to any of claims 1 - 3,
wherein the cell to be transfected is selected from the group consisting of cardiomyocytes, endothelial cells, sensory neurons, motor neurons, CNS neurons, astrocytes, glial cells, Schwann cells, mast cells, eosinophils, smooth muscle cells, skeletal muscle cells, pericytes, lymphocytes, tumor cells, monocytes, macrophages, foamy macrophages, dentritic cells, granulocytes, melanocytes, keratinocytes, synovial cells/synovial fibroblasts and epithelial cells.

**9.** The method according to any of claims 1 - 3,
wherein the capture molecules are deposited by physical deposition method on the substrate; or
wherein the capture molecules are synthesized in situ on the substrate in predefined locations; or
wherein the capture molecules are polynucleotides and are unique for each of the genes to be detected and/or quantified on the array, preferably wherein the length of the polynucleotides is comprised between 15 and 1000 nucleotides; or
wherein the amount of capture nucleotide sequence bound to the surface of the substrate is superior to 3 fmoles per cm$^2$ of solid support surface.

**10.** The method according to any of claims 1 - 3,
wherein the sample component derived from the transfected cell(s) is RNA transcripts or nucleic acids derived from said RNA transcripts; preferably wherein nucleic acids derived from said RNA transcripts is cDNA; or
wherein sample and the control experimental conditions are analyzed on the same substrate having two identical array; orwherein the sample to be tested and the control are analyzed on arrays bearing the same capture molecules for the said analyzed genes; or
wherein the measurement on the array of the genes expressed in the cells are quantitative with coefficient of variation of the data lower than 80% even lower than 25% even lower than 15%.

**11.** A kit for the determination and/or quantification of the effects of siRNA transfection in cell(s) by the determination of non-specific RNAi effects comprising :

an array, having fixed on a substrate a maximum of 2999 different capture molecules specific of nucleic acids or proteins belonging to or representative of each of the following 3 non-specific effect functions: apoptosis, DNA repair/synthesis and interferon response wherein the array allows the detection of at least 20 different said nucleic acids or proteins, and
optionally buffers and labels.

**12.** A kit for the determination and/or quantification of the effects of siRNA transfection in cell(s) by the determination specific and non-specific RNAi effects, comprising :

an array, having fixed on a substrate a maximum of 2999 different capture molecules specific of nucleic acids or proteins belonging to or representative of each of the fol lowing 3 non-specific effect functions: apoptosis, DNA repair/synthesis and interferon response and at least one nucleic acid or protein, belonging to or representative of at least 4 of the following vital cellular functions: cell adhesion, cell cycle, growth factors and cytokines, cell signalling/receptor, chromosomal processing, intermediate metabolism, extracellular matrix, cell structure, protein metabolism, oxidative metabolism, transcription and housekeeping genes, wherein the array allows the detection of at least 20 different said nucleic acids or proteins, and
optionally buffers and labels.

**13.** A method for screening of siRNA for the absence of non-specific side effects comprising the steps of:

providing an array, having fixed on a substrate a maximum of 2999 different capture molecules specific of nucleic acids or proteins belonging to or representative for each of the following 3 non-specific effect functions: apoptosis, DNA repair/synthesis and interferon response wherein the array allows the detection of at least 20 different said nucleic acids or proteins,
contacting a sample component derived from said transfected cell(s) with the array, detecting and/or quantifying the intensity of the signal on said capture molecules, comparing the transcriptome or proteome of said transfected cell(s) with at least one reference or control condition,
wherein the pattern and/or the intensity of the binding events is indicative of the non-specific side effects of the presence of siRNA in said transfected cell(s).

**14.** A method for improving the design of the sequence of the siRNA comprising the steps of:

providing an array, having fixed on a substrate a maximum of 2999 different capture molecules specific of nucleic acids or proteins belonging to or representative for each of the following 3 non-specific effect functions: apoptosis, DNA repair/synthesis and interferon response wherein the array allows the detection of at least 20 different said nucleic acids or proteins,
transfecting cells with siRNA having different sequence and/or different length, contacting a sample component derived from said transfected cell(s) with the array, detecting and/or quantifying the intensity of the signal on said capture molecules, comparing the transcriptome or proteome of said transfected cell(s) with at least one reference or control condition, comprising the transcriptome or proteome of cells having been transfected with different siRNA's,
wherein the difference in pattern and/or the intensity of the binding events is indicative of the effectiveness of a given siRNA.

**European Patent Office**

## EUROPEAN SEARCH REPORT

Application Number

EP 05 02 3684

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SLEDZ C A ET AL: "ACTIVATION OF THE INTERFERON SYSTEM BY SHORT-INTERFERING RNAS" NATURE CELL BIOLOGY, MACMILLAN PUBLISHERS, GB, vol. 5, no. 9, September 2003 (2003-09), pages 834-839, XP009043516 ISSN: 1465-7392 * the whole document * | 1-14 | C12Q1/68 |
| X,D | PERSENGIEV STEPHAN P ET AL: "Nonspecific, concentration-dependent stimulation and repression of mammalian gene expression by small interfering RNAs (siRNAs)." RNA (COLD SPRING HARBOR), vol. 10, no. 1, January 2004 (2004-01), pages 12-18, XP002369414 ISSN: 1355-8382 * the whole document * | 1-14 | |
| A,D | SCACHERI PETER C ET AL: "Short interfering RNAs can induce unexpected and divergent changes in the levels of untargeted proteins in mammalian cells." PROCEEDINGS OF THE NATIONAL ACADEMY OF SCIENCES OF THE UNITED STATES OF AMERICA, vol. 101, no. 7, 17 February 2004 (2004-02-17), pages 1892-1897, XP002369415 ISSN: 0027-8424 * abstract * * page 1896, column 1, paragraph 2 - column 2, paragraph 3 * | 1-14 | **TECHNICAL FIELDS SEARCHED (IPC)**<br><br>C12Q |
| A | US 6 183 968 B1 (BANDMAN OLGA ET AL) 6 February 2001 (2001-02-06) * column 1, line 54 - column 2, line 34 * | 1-14 | |

-/--

The present search report has been drawn up for all claims

| Place of search | Date of completion of the search | Examiner |
|---|---|---|
| Munich | 23 February 2006 | Bradbrook, D |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons

& : member of the same patent family, corresponding
　　document

EPO FORM 1503 03.82 (P04C01)

**European Patent Office**

**EUROPEAN SEARCH REPORT**

Application Number

EP 05 02 3684

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LACROIX M ET AL: "A LOW-DENSITY DNA MICROARRAY FOR ANALYSIS OF MARKERS IN BREAST CANCER" INTERNATIONAL JOURNAL OF BIOLOGICAL MARKERS, WICHTIG EDITORE, MILAN, IT, vol. 17, no. 1, 2002, pages 5-23, XP008010290 ISSN: 0393-6155 * the whole document * | 1-14 | |
| P,X | EP 1 477 571 A (EPPENDORF AG) 17 November 2004 (2004-11-17) * the whole document * | 1-14 | |

TECHNICAL FIELDS SEARCHED (IPC)

The present search report has been drawn up for all claims

| Place of search | Date of completion of the search | Examiner |
|---|---|---|
| Munich | 23 February 2006 | Bradbrook, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 02 3684

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2006

| Patent document cited in search report | | Publication date | Patent family member(s) | | Publication date |
|---|---|---|---|---|---|
| US 6183968 | B1 | 06-02-2001 | NONE | | |
| EP 1477571 | A | 17-11-2004 | US<br>US | 2004229225 A1<br>2005208518 A1 | 18-11-2004<br>22-09-2005 |

EPO FORM P0459

For more details about this annex : see Official Journal of the European Patent Office, No. 12/82